# EUROPEAN PATENT APPLICATION

(11) **EP 4 428 149 A1**
(43) Date of publication of application: **11.09.2024**
(21) Application number: 22890426.4
(22) Date of filing: 04.11.2022
(51) Int. Cl.: C07K 16/18, A61K 39/00

(54) **RESISTIN-SPECIFIC ANTIBODY AND USE THEREOF**

(30) Priority: 05.11.2021 KR 20210151465
(71) Applicant: Seoul National University Hospital, Seoul 03080 (KR); Y-Biologics Inc., Daejeon 34014 (KR)
(72) Inventor: KIM, Hyo-Soo, Seoul 04401 (KR); JANG, Hyun-Duk, Seoul 03319 (KR); PARK, Bum-Chan, Daejeon 34014 (KR); YOON, Jae Bong, Daejeon 34014 (KR); PARK, Jae Eun, Daejeon 34014 (KR); YANG, So Young, Daejeon 34014 (KR); JEON, Eun Young, Daejeon 34014 (KR); KIM, Soo Young, Daejeon 34014 (KR); LEE, Ji Su, Daejeon 34014 (KR); LEE, Jae Min, Daejeon 34014 (KR); LEE, Dong Jung, Daejeon 34014 (KR); SONG, Ji Ahn, Daejeon 34014 (KR)
(74) Representative: Mewburn Ellis LLP
(86) International application number: PCT/KR2022/017192
(87) International publication number: WO 2023/080695

(57) **Abstract**

The present invention relates to an antibody against resistin and use thereof and, more specifically, to a resistin antibody or antigen-binding fragment thereof that inhibits the activity of resistin by blocking resistin/CAP1 binding, a nucleic acid encoding same, a vector comprising the nucleic acid, a cell transformed with the vector, a method for preparing the antibody or antigen-binding fragment thereof, an antibody-drug conjugate comprising the antibody or antigen-binding fragment thereof, a bi- or multi-specific antibody, a chimeric antigen receptor, an immune cell containing same, and a composition for the prevention or treatment of diseases that can be treated through the inhibition of resistin activity, the composition comprising same. A novel antibody or antigen-binding fragment thereof that binds to resistin, according to the present invention, blocks resistin/CAP1 binding by binding to resistin, thereby inhibiting the activity of resistin, and thus is useful for the development of therapeutic agents for various diseases associated with resistin.

## Description

### TECHNICAL FIELD

The present invention relates to an antibody against resistin and use thereof, and more specifically, a resistin antibody or antigen-binding fragment thereof that inhibits the activity of resistin by blocking resistin/CAP1 binding, a nucleic acid encoding the same, a vector comprising the nucleic acid, a cell transformed with the vector, a method for preparing the antibody or antigen-binding fragment thereof, an antibody-drug conjugate comprising the antibody or antigen-binding fragment thereof, a bi- or multi-specific antibody, a chimeric antigen receptor, an immune cell comprising the same, and a composition for the prevention or treatment of diseases that can be treated by inhibiting the resistin activity.

### BACKGROUND ART

Resistin is a cytokine that was first identified as a mediator inducing insulin resistance in obese mice. It belongs to a family of cysteine-rich proteins, also known as resistin-like molecules (RELMs), and is relevant to the regulation of inflammatory processes. In addition, murine resistin is known to be related to the obesity-mediated insulin resistance and the development of and type 2 diabetes (Li et al, Endocrine 35:243-251, 2009; Nakata et al, Biochem Biophys Res Commun. 353:1046-1051, 2007; Steppan et al, Nature 409:307-312, 2001).

In fact, the protein sequence of rat and that of human resistin are only about 60% identical, and rodent resistin is first expressed and secreted primarily in mature adipocytes, whereas human resistin is secreted primarily in peripheral blood mononuclear cells (PBMCs) and macrophages, of leukocytes. Many studies have shown that the role of resistin is different in humans and rodents.

The actions of human resistin have been reported to promote the recruitment of immune cells and induce the secretion of pro-inflammatory factors, and there is evidence that it induces inflammatory diseases and atherosclerosis apart from promoting insulin resistance (Bokarewa et al, J Immunol. 174:5789-5795, 2005; Silswal et al, Biochem Biophys Res Commun. 334:1092-1101, 2005; Burnett et al, Atherosclerosis 182:241-248, 2005; Jung et al, Cardiovasc. Res 69:76-85, 2006; Reilly et al, Circulation 111:932-939, 2005). In addition, resistin, which is present in both murine and human atherosclerotic lesions, is known as an inflammatory marker of atherosclerosis in humans and to promote atherosclerosis by activating monocytes (Cho et al, J Am Coll Cardiol 57:99-109, 2011). Thus, human resistin may be a key factor in stimulating monocytes that lead to atherosclerosis.

The mechanism by which human resistin induces inflammation appears to be activation of the nuclear factor kappa B (NF-κB) transcription factor, but the signaling pathway exhibiting the pro-inflammatory effects of resistin remains unclear, and the role of resistin in cancer remains unclear.

The inventors of the present invention were the first in the world to discover CAP1 (adenylyl cyclaseassociated protein 1), a receptor that directly interacts with human resistin (Lee S et al, Cell Metabolism, 19(3): 484-97, 2014). Thus, by utilizing CAP1, the receptor that mediates the action of human resistin, it is possible to develop therapeutics for diseases caused by resistin.

Accordingly, the present inventors have made a good faith effort to treat diseases caused by resistin, such as cancer, by blocking resistin/CAP1 binding, and thus have produced a resistin-specific antibody that binds to resistin with high affinity, and have confirmed that the anti-resistin antibody has high therapeutic effect on diseases that can be treated by inhibitory activity against resistin/CAP1 binding and inhibiting the activity of resistin, and have completed the present invention.

The information disclosed in this Background section is provided only for enhancement of understanding of the background of the present invention, and therefore it may not include information that forms the prior art that is already obvious to those skilled in the art.

### SUMMARY OF THE INVENTION

It is an object of the present invention to provide a novel antibody to resistin or antigen-binding fragment thereof.

It is another object of the present invention to provide a nucleic acid encoding the antibody or antigen-binding fragment thereof.

It is still another object of the present invention to provide a vector comprising the nucleic acids, a cell transformed with the vector, and a method for preparing the antibody or antigen-binding fragment thereof using the vector.

It is yet another object of the present invention to provide an antibody-drug conjugate or bi- or multi-specific antibody comprising the antibody or antigen-binding fragment thereof.

It is further another object of the present invention to provide a chimeric antigen receptor comprising the antibody or antigen-binding fragment thereof, an immune cell comprising the chimeric antigen receptor.

It is still yet another object of the present invention to provide a pharmaceutical composition for the prevention or treatment of a disease that can be treated by inhibition of the activity of resistin, and a method for preventing or treating a disease that can be treated by inhibition of the activity of resistin, comprising the antibody or antigen-binding fragment thereof, the antibody-drug conjugate, the bi- or multi-specific antibody, or the chimeric antigen receptor; the use of the antibody or antigen-binding fragment thereof, the antibody-drug conjugate, the bi- or multi-specific antibody or the chimeric antigen receptor for the prevention or treatment of a disease that can be treated by inhibition of the activity of resistin; and the use of the antibody or antigen-binding fragment thereof, the antibody-drug conjugate, the bi- or multi-specific antibody or the chimeric antigen receptor in the manufacture of a medicament for preventing or treating a disease that can be treated by inhibition of the activity of resistin.

To accomplish the above objectives, the present invention provides an antibody that specifically binds to resistin or antigen-binding fragment thereof, comprising a heavy chain variable region comprising CDR1 comprising an amino acid sequence of SEQ ID NO: 1, CDR2 comprising an amino acid sequence of SEQ ID NO: 2, and CDR3 comprising an amino acid sequence of SEQ ID NO: 3; and
a light chain variable region comprising CDR1 comprising an amino acid sequence selected from the group consisting of SEQ ID NOs: 4 to 11, CDR2 comprising an amino acid sequence selected from the group consisting of SEQ ID NOs: 12 to 19, and CDR3 comprising an amino acid sequence selected from the group consisting of SEQ ID NOs: 20 to 27.

The present invention also provides a nucleic acid encoding a heavy chain variable region of the antibody or antigen-binding fragment thereof.

The present invention also provides a nucleic acid encoding a light chain variable region of the antibody or antigen-binding fragment thereof.

The present invention also provides a vector comprising the nucleic acid.

The present invention also provides cells transformed with the vector.

The present invention also provides a method of producing the antibody or antigen-binding fragment thereof, comprising (a) culturing the cells; and (b) recovering the antibody or antigen-binding fragment thereof from the cultured cells.

The present invention also provides an antibody-drug conjugate or bi- or multi-specific antibody comprising the antibody or antigen-binding fragment thereof.

The present invention also provides a chimeric antigen receptor comprising the antibody or antigen-binding fragment thereof, or an immune cell comprising the chimeric antigen receptor.

The present invention also provides a pharmaceutical composition for the prevention or treatment of a disease that can be treated by inhibiting the activity of resistin, comprising the antibody or antigen-binding fragment thereof, the antibody-drug conjugate, the bi- or multi-specific antibody, or the chimeric antigen receptor.

The present invention also provides a method of preventing or treating a disease that can be treated by inhibiting the activity of resistin using the antibody or antigen-binding fragment thereof, antibody-drug conjugate, bi- or multispecific antibody, or chimeric antigen receptor; the use of the antibody or antigen-binding fragment thereof, the antibody-drug conjugate, the bi- or multi-specific antibody or the chimeric antigen receptor for the prevention or treatment of a disease that can be treated by inhibition of the activity of resistin; and the use of the antibody or antigen-binding fragment thereof, the antibody-drug conjugate, the bi- or multi-specific antibody or the chimeric antigen receptor in the manufacture of a medicament for preventing or treating a disease that can be treated by inhibition of the activity of resistin.

### BRIEF DESCRIPTION OF DRAWINGS

FIG. 1 shows the results confirming the purity by analyzing RETN-C-Fc antigen and RETN-flag-His antigen with SDS-PAGE after primary purification.
FIG. 2a shows the results of a polyphage ELISA of the scFv phage pool using RETN-flag-His as an antigen in the first round of biopanning.
FIG. 2b shows the results of a polyphage ELISA of the scFv phage pool using RETN-C-Fc as an antigen in the first round of biopanning.
FIG. 3a shows the results of an antigen-specific binding analysis of monoclones obtained in the first round of biopanning using RETN-flag-His as an antigen.
FIG. 3b shows the results of an antigen-specific binding analysis of monoclones obtained in the first round of biopanning using RETN-C-Fc as the antigen.
FIG. 4 shows the results of an antigen-receptor binding inhibition ability analysis of 16 proteins obtained from the first screening.
FIG. 5 shows the results of antigenic affinity analysis of the 4 clones using OCTET.
FIG. 6 shows the results of antigen-specific binding analysis of 11 monoclonals obtained from the second screening.
FIG. 7a shows the results of antigen-specific binding analysis of 10 monoclonals obtained from the first biopanning of the third screening.
FIG. 7b shows the results of antigen-specific binding analysis of 9 monoclonals obtained from the second biopanning of the third screening.
FIG. 8 shows 5 antibodies (11G01, 27A04, 32B05, 32E06 and 32G09) with good antigen-receptor binding inhibition ability effect.
FIG. 9a shows the results of a specific binding ELISA analysis for RETN-flag-His antigen of 20 clones.
FIG. 9b shows the results of a non-specific binding ELISA analysis of 20 clones.
FIG. 10 shows the antigenic affinity analysis of 6 clones using OCTET.
FIG. 11 is a schematic of the preparation of a light chain shuffling library (LC shuffling library).
FIG. 12 shows polyphage ELISA results of a pool of scFv phages obtained by biopanning with a light chain shuffling library.
FIG. 13 shows the change in productivity of 18 antibody optimized clones relative to the parent antibody RETN-5A2 clone.
FIG. 14 shows the results of SDS-PAGE analysis of 18 antibody optimized clones.
FIG. 15a shows an ELISA analysis of the binding of 18 optimized antibodies to RETN-flag-His antigen.
FIG. 15b shows the non-specific binding analysis of 18 optimized antibodies using ELISA.
FIG. 15c shows ELISA analysis of the binding of 13 optimized antibodies to intact form resistin antigen.
FIG. 16 shows ELISA analysis of the cross-species binding reactions of 8 optimized antibodies to murine resistin antigen.
FIG. 17a is a schematic of an antigen-receptor competitive enzyme immunoassay.
FIG. 17b shows an antigen-receptor competitive enzyme immunoassay to evaluate the ability inhibiting antigen-receptor binding of 18 optimized antibodies.
FIG. 18 shows the antigen-receptor binding inhibition ability analyzed by pull down assay.
FIG. 19 shows the antigen affinity analysis of 7 clones of optimized antibodies using OCTET.
FIG. 20 shows NF-κB activation by resistin, as measured by phosphorylation of p65, after treatment of breast cancer cells with resistin.
FIG. 21 shows the pharmacokinetics of 5A2 antibody and hlgG control administered to a mouse.
FIG. 22 is a diagram illustrating inhibition of migration of breast cancer cells in vitro by a 5A2 antibody.
FIG. 23 is a diagram illustrating inhibition of metastasis of breast cancer cells in vivo by a 5A2 antibody.
FIG. 24 is a schematic of a mouse NASH model experiment to determine the effectiveness of a 5A2 antibody.
FIG. 25 shows H&E analysis of the suppression of fatty liver when a mouse is administered with a 5A2 antibody.
FIG. 26 shows the suppression of fatty liver when a mouse is administered with a 5A2 antibody, as confirmed by an oil-red-o assay.
FIG. 27 is a graph showing insulin sensitivity when a mouse is administered with a 5A2 antibody.
FIG. 28 is a graph showing the reduction in fasting blood glucose level when a mouse is administered with a 5A2 antibody.
FIG. 29 is a graph showing the reduction in LDL-cholesterol when a mouse is administered with a 5A2 antibody.
FIG. 30 is a graph showing the reduction in liver triglycerides when a mouse is administered with a 5A2 antibody.
FIG. 31 is a schematic of a mouse IBD model experiment to determine the effectiveness of an RETN-5A2_LS 2F11 antibody.
FIG. 32 is a diagram illustrating the inhibition of mouse weight loss by administration of RETN-5A2_LS 2F11 antibody in an IBD mouse model.
FIG. 33 is a diagram illustrating the inhibition of Disease Activity Score increase by administration of RETN-5A2_LS 2F11 antibody in an IBD mouse model.
FIG. 34 is a diagram illustrating the gut length protection by administration of RETN-5A2_LS 2F11 antibody in an IBD mouse model.
FIG. 35 shows the results of histological scoring when an IBD mouse model is administered with RETN-5A2_LS 2F11 antibody.

### DETAILED DESCRIPTION AND PREFERRED EMBODIMENTS OF THE INVENTION

Unless defined otherwise, all technical and scientific terms used herein have the same meanings as appreciated by those skilled in the field to which the present invention pertains. In general, the nomenclature used herein is well-known in the art and is ordinarily used.

The present inventors have used a biopanning method to screen a novel resistin human antibody that has high affinity for human resistin and excellent resistin antigen-receptor binding inhibition ability, and have confirmed that the screened resistin human antibody exhibits anti-cancer effects that inhibit the migration and metastasis of cancer cells.

Accordingly, in one aspect, the present invention provides an antibody that specifically binds to resistin or antigen-binding fragment thereof, comprising a heavy chain variable region comprising CDR1 comprising an amino acid sequence of SEQ ID NO: 1, CDR2 comprising an amino acid sequence of SEQ ID NO: 2, and CDR3 comprising an amino acid sequence of SEQ ID NO: 3; and
a light chain variable region comprising CDR1 comprising an amino acid sequence selected from the group consisting of SEQ ID NOs: 4 to 11, CDR2 comprising an amino acid sequence selected from the group consisting of SEQ ID NOs: 12 to 19, and CDR3 comprising an amino acid sequence selected from the group consisting of SEQ ID NOs: 20 to 27.

In the present invention, the light chain variable region is:
a light chain variable region comprising CDR1 comprising the amino acid sequence of SEQ ID NO: 4, CDR2 comprising the amino acid sequence of SEQ ID NO: 12, and CDR3 comprising the amino acid sequence of SEQ ID NO: 20;
a light chain variable region comprising CDR1 comprising the amino acid sequence of SEQ ID NO: 5, CDR2 comprising the amino acid sequence of SEQ ID NO: 13, and CDR3 comprising the amino acid sequence of SEQ ID NO: 21;
a light chain variable region comprising CDR1 comprising the amino acid sequence of SEQ ID NO: 6, CDR2 comprising the amino acid sequence of SEQ ID NO: 14, and CDR3 comprising the amino acid sequence of SEQ ID NO: 22;
a light chain variable region comprising CDR1 comprising the amino acid sequence of SEQ ID NO: 7, CDR2 comprising the amino acid sequence of SEQ ID NO: 15, and CDR3 comprising the amino acid sequence of SEQ ID NO: 23;
a light chain variable region comprising CDR1 comprising the amino acid sequence of SEQ ID NO: 8, CDR2 comprising the amino acid sequence of SEQ ID NO: 16, and CDR3 comprising the amino acid sequence of SEQ ID NO: 24;
a light chain variable region comprising CDR1 comprising the amino acid sequence of SEQ ID NO: 9, CDR2 comprising the amino acid sequence of SEQ ID NO: 17, and CDR3 comprising the amino acid sequence of SEQ ID NO: 25;
a light chain variable region comprising CDR1 comprising an amino acid sequence of SEQ ID NO: 10, CDR2 comprising an amino acid sequence of SEQ ID NO: 18, and CDR3 comprising an amino acid sequence of SEQ ID NO: 26; or
a light chain variable region comprising CDR1 comprising an amino acid sequence of SEQ ID NO: 11, CDR2 comprising an amino acid sequence of SEQ ID NO: 19, and CDR3 comprising an amino acid sequence of SEQ ID NO: 27.

Furthermore, in the present invention, the heavy chain variable region preferably comprises the amino acid sequence of SEQ ID NO: 28, and the light chain variable region preferably comprises an amino acid sequence selected from the group consisting of SEQ ID NOs: 32, 34, 36, 38, 40, 42, 44 and 46. More preferably, the antibody or antigen-binding fragment thereof of the present invention comprise, but are not limited to, a heavy chain variable region comprising an amino acid sequence of SEQ ID NO: 28; and a light chain variable region comprising an amino acid sequence selected from the group consisting of SEQ ID NOs: 32, 34, 36, 38, 40, 42, 44 and 46.

The heavy chain variable region CDR sequences (SEQ ID NOs: 1, 2 and 3) of the antibodies that specifically bind to resistin of the present invention are shown in Table 1 below.

The light chain variable region CDR sequences (SEQ ID NOs: 4 to 27) of the antibodies that specifically bind to the resistin of the present invention are shown in Table 2 below. SEQ ID NOs: 11, 19 and 27 represent the sequences of the light chain variable region CDR1, 2 and 3 of the resistin 5A2 parent antibody.

The amino acid sequence of the heavy chain variable region (SEQ ID NO: 28) and the amino acid sequence of the heavy chain constant region (SEQ ID NO: 29) of an antibody that specifically binds to resistin of the present invention are shown in Table 3 below.

Furthermore, the amino acid sequences of the light chain variable region (SEQ ID NOs: 32, 34, 36, 38, 40, 42, 44 and 46) and the amino acid sequences of the light chain constant region (SEQ ID NOs: 33, 35, 37, 39, 41, 43, 45 and 47) of an antibody that specifically binds to the resistin of the present invention are shown in Table 5 below. SEQ ID No: 46 represents the amino acid sequence of the light chain variable region of the resistin 5A2 parent antibody, and SEQ ID No: 47 represents the amino acid sequence of the light chain constant region of the resistin 5A2 parent antibody.

**[Table 1]**

| CDR sequences of heavy chain variable regions | | | |
|---|---|---|---|
| NAME | Heavy chain variable region CDR | | |
| | CDR1 | CDR2 | CDR3 |
| SEQ ID NO. 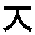 | 1 | 2 | 3 |
| RETN-5A2 | SYAIS | GIIPIFGAADYAQKFKG | GPDYYGSFDP |

**[Table 2]**

| CDR sequences of light chain variable regions | | | |
|---|---|---|---|
| NAME | Light chain variable region CDR | | |
| | CDR1 | CDR2 | CDR3 |
| SEQ ID NO. | 4 | 12 | 20 |
| RETN-5A2_LS_1F11 | KSSQSVLYSSNNKNYLA | WASTRES | QQYYSEPL |
| SEQ ID NO. | 5 | 13 | 21 |
| RETN-5A2_LS_2B02 | RASQSVRGSYLA | GTSSRAP | QQRGDWLYT |
| SEQ ID NO. | 6 | 14 | 22 |
| RETN-5A2_LS_2D07 | TGTSSDIGRYNRVS | DVSNRPS | SAYTRSSTWV |
| SEQ ID NO. | 7 | 15 | 23 |
| RETN-5A2_LS_2F11 | RASQSIGTYLN | DASSLED | QQSYTMPLYT |
| SEQ ID NO. | 8 | 16 | 24 |
| RETN-5A2_LS_2G03 | QASQHVSSYLN | AASNLQS | QQYDSYSSVYT |
| SEQ ID NO. | 9 | 17 | 25 |
| RETN-5A2_LS_2E10 | RTSQTITNYLN | DASNLET | QQSYNPGYT |
| SEQ ID NO. | 10 | 18 | 26 |
| RETN-5A2_LS_2H10 | TGSSSDVGGYNYVT | DVTKRPS | SSYSSSTFYV |
| SEQ ID NO. | 11 | 19 | 27 |
| RETN-5A2 | RASQYIGTYLA | GASILQN | QQADSFPLT |

**[Table 3]**

| Heavy chain amino acid sequences | | |
|---|---|---|
| NAME | Variable region amino acid sequence | Constant region amino acid sequence |
| RETN-5A2 | QVQLVQSGAEGRKPGSSV KVSCKVSGDTFSSYAISWV RQAPGQGLEWMGGIIPIFG AADYAQKFKGRVTISADEST STAYMELSSLRSEDTAVYY CAKGPDYYGSFDPWGQGT LITVSS (SEQ ID NO: 28) | ASTKGPSVFPLAPSSKSTSGGTAALGCLVKDYFPEPV TVSWNSGALTSGVHTFPAVLQSSGLYSLSSVVTVPSS SLGTQTYICNVNHKPSNTKVDKKVEPKSCDKTHTCPP CPAPELLGGPSVFLFPPKPKDTLMISRTPEVTCVVVDV SHEDPEVKFNWYVDGVEVHNAKTKPREEQYNSTYRV VSVLTVLHQDWLNGKEYKCKVSNKALPAPIEKTISKAK GQPREPQVYTLPPSREEMTKNQVSLTCLVKGFYPSDI AVEWESNGQPENNYKTTPPVLDSDGSFFLYSKLTVD KSRWQQGNVFSCSVMHEALHNHYTQKSLSLSPGK (SEQ ID NO: 29) |

**[Table 4]**

| Heavy chain nucleotide sequences | | |
|---|---|---|
| NAME | Variable region nucleotide sequence | Constant region nucleotide sequence |
| RETN-5A2 | CAGGTGCAGCTGGTGCAGT CTGGGGCTGAGGGGAGGA AGCCTGGGTCCTCGGTGAA GGTCTCCTGCAAGGTTTCT GGAGACACCTTCAGCAGTT ATGCTATCAGCTGGGTGCG ACAGGCCCCTGGACAAGGG CTTGAGTGGATGGGAGGGA TCATCCCTATCTTTGGTGCA GCAGACTACGCACAGAAGT TCAAGGGCAGAGTCACGAT TAGTGCGGACGAATCCACG AGCACAGCCTACATGGAGC TGAGCAGCCTGAGATCTGA GGACACGGCCGTGTATTAC TGTGCGAAAGGACCGGATT ATTATGGTTCGTTTGACCCC TGGGGCCAGGGAACCCTGA TCACCGTCTCCTCA (SEQ ID NO: 30) | GCTAGCACCAAGGGCCCATCGGTCTTCCCCCTG GCACCCTCCTCCAAGAGCACCTCTGGGGGCACA GCGGCCCTGGGCTGCCTGGTCAAGGACTACTTC CCCGAACCGGTGACGGTGTCGTGGAACTCAGGC GCCCTGACCAGCGGCGTGCACACCTTCCCGGCT GTCCTACAGTCCTCAGGACTCTACTCCCTCAGCA GCGTGGTGACCGTGCCCTCCAGCAGCCTGGGCA CCCAGACCTACATCTGCAACGTGAATCACAAGCC CAGCAACACCAAGGTGGACAAGAAAGTTGAGCC CAAATCTTGTGACAAAACTCACACATGCCCACCG TGCCCAGCACCTGAACTCCTGGGGGGACCGTCA GTCTTCCTCTTCCCCCCAAAACCCAAGGACACCC TCATGATCTCCCGGACCCCTGAGGTCACATGCGT GGTGGTGGACGTGAGCCACGAAGACCCTGAGGT CAAGTTCAACTGGTACGTGGACGGCGTGGAGGT GCATAATGCCAAGACAAAGCCGCGGGAGGAGCA GTACAACAGCACGTACCGTGTGGTCAGCGTCCTC ACCGTCCTGCACCAGGACTGGCTGAATGGCAAG GAGTACAAGTGCAAGGTCTCCAACAAAGCCCTCC CAGCCCCCATCGAGAAAACCATCTCCAAAGCCAA AGGGCAGCCCCGAGAACCACAGGTGTACACCCT GCCCCCATCCCGGGAGGAGATGACCAAGAACCA GGTCAGCCTGACCTGCCTGGTCAAAGGCTTCTAT CCCAGCGACATCGCCGTGGAGTGGGAGAGCAAT GGGCAGCCGGAGAACAACTACAAGACCACGCCT CCCGTGCTGGACTCCGACGGCTCCTTCTTCCTCT ACAGCAAGCTCACCGTGGACAAGAGCAGGTGGC AGCAGGGGAACGTCTTCTCATGCTCCGTGATGCA |
| | | TGAGGCTCTGCACAACCACTACACGCAGAAGAG CCTCTCCCTGTCTCCGGGTAAA (SEQ ID NO: 31) |

**[Table 5]**

| Light chain amino acid sequences | | |
|---|---|---|
| NAME | VL | CL |
| RETN-5A2_LS_ 1F11 | DIQMTQSPDSLAVSLERGATINCKSSQSVL YSSNNKNNYLAWYQHKPGQPPRLIYWAST RESGVPDRFSGSGSGTDFTLTINGLQAED VAVYYCQQYYSSEPLFGPGTKVDIK (SEQ ID NO: 32) | RSVAAPSVFIFPPSDEQLKSGTASVVCLLN NFYPREAKVQWKVDNALQSGNSQESVTE QDSKDSTYSLSSTLTLSKADYEKHKVYACE VTHQGLSSPVTKSFNRGEC (SEQ ID NO: 33) |
| RETN-5A2_LS_ 2B02 | DIQMTQSPGTLSLSAGERATLSCRASQSV RGSYSLAWYQQKPGQAPRLLIYGTSSRAP GIPDRFSGSGSGTDFTLTISSLEPEDFAVY YCQQRGDWLYTFGQGTKVEIK (SEQ ID NO: 34) | RSVAAPSVFIFPPSDEQLKSGTASVVCLLN NFYPREAKVQWKVDNALQSGNSQESVTE QDSKDSTYSLSSTLTLSKADYEKHKVYACE VTHQGLSSPVTKSFNRGEC (SEQ ID NO: 35) |
| RETN-5A2_LS_ 2D07 | QSALTQPASVSGSPGQSVTISCTGTSSDIG RYNRRVSWYQHHPGKAPKLIIYDVSNRPS GVSNRFSGSKSGNTASLTISGLQAGDESD YYCSAYTRSSTWVFGGGGTKLTVL (SEQ ID NO: 36) | RSVAAPSVFIFPPSDEQLKSGTASVVCLLN NFYPREAKVQWKVDNALQSGNSQESVTE QDSKDSTYSLSSTLTLSKADYEKHKVYACE VTHQGLSSPVTKSFNRGEC (SEQ ID NO: 37) |
| RETN-5A2_LS_ 2F11 | DIQMTQSPSSLSASVGDRVTITCRASQSIG TYLNWYQQKPGKAPKLLLIYDASSLEDGVP SRFSGSGSGTDFTLTISSLQPEDSATYYCQ QSYTMPLYTFGQGTKLEIK (SEQ ID NO: 38) | RSVAAPSVFIFPPSDEQLKSGTASVVCLLN NFYPREAKVQWKVDNALQSGNSQESVTE QDSKDSTYSLSSTLTLSKADYEKHKVYACE VTHQGLSSPVTKSFNRGEC (SEQ ID NO: 39) |
| RETN-5A2_LS_ 2G03 | DIQMTQSPSSLSASVGDRVTITCQASQHV | RSVAAPSVFIFPPSDEQLKSGTASVVCLLN |
| | SSYLNWYQQKPGKAPKLLLIYAASNLQSG VPSRFSGSGSGTDFTLTISSLQPDDFATYY CQQYDSYSSVYTFGPGTKLDIK (SEQ ID NO: 40) | NFYPREAKVQWKVDNALQSGNSQESVTE QDSKDSTYSLSSTLTLSKADYEKHKVYACE VTHQGLSSPVTKSFNRGEC (SEQ ID NO: 41) |
| RETN-5A2_LS_ 2E10 | DIQMTQSPSSLSASVGDRVTITCRTSQTIT NYLNWYQQKPGRAPKLLIFDASNLETGVP SRFSGSGSGTDFTLTISSLQPEDFATYFCQ QSYNPGYTFGQGTKLEIK (SEQ ID NO: 42) | RSVAAPSVFIFPPSDEQLKSGTASVVCLLN NFYPREAKVQWKVDNALQSGNSQESVTE QDSKDSTYSLSSTLTLSKADYEKHKVYACE VTHQGLSSPVTKSFNRGEC (SEQ ID NO: 43) |
| RETN-5A2_LS_ 2H10 | NFMLTQPASVSGSPGQSITISCTGSSSDSD VGGGYNYVTWYQQHPGKAPQLIIYDVTKR PSGVSNRFSGSKSGNSASLTISGLQAEDE ADYYCSSYSSSSTFYVFGTGTKVTVL (SEQ ID NO: 44) | RSVAAPSVFIFPPSDEQLKSGTASVVCLLN NFYPREAKVQWKVDNALQSGNSQESVTE QDSKDSTYSLSSTLTLSKADYEKHKVYACE VTHQGLSSPVTKSFNRGEC (SEQ ID NO: 45) |
| RETN-5A2_LC | DIQMTQSPSFLSASVGDRVTITCRASQYIG TYLAWYQQQPGKAPKLLLIYGASILQNGVP SRFSGSASGTEFTLTISSLQPEDFATYYCQ QADSFPLTFGGGTKVDIK (SEQ ID NO: 46) | RSVAAPSVFIFPPSDEQLKSGTASVVCLLN NFYPREAKVQWKVDNALQSGNSQESVTE QDSKDSTYSLSSTLTLSKADYEKHKVYACE VTHQGLSSPVTKSFNRGEC (SEQ ID NO: 47) |

**[Table 6]**

| Light chain nucleotide sequences | | |
|---|---|---|
| NAME | VL | CL |
| RETN-5A2_LS_ 1F11 | GACATCCAGATGACCCAGTCTCCAGACT CCCTGGCTGTGTCTCTGGGCGAGAGGG CCACCATCAACTGCAAGTCCAGCCAGAG CGTTTTATACAGCTCCAACAATAAGAACT ACTTAGCTTGGTACCAACACAAACCGGG | AGATCTGTGGCTGCCTGCACCATCTGCT TCATCTTCCCGCCATCTGATGAGCAGTT GAAATCTGGAAACTGCCTCTGTTGTGCC TGCCTGCTGAATAAACTTCTATCCCAGAG AGGCCAAAGTACAGTGGAAGGTGATAAC |
| | ACAACCTCCTAGGTTGCTCATTTACTGG GCATCCACCCGGGAATCCGGGGTCCCC GATCGATTCAGTGGCAGCGGGTCTGGG ACAGATTTCACTCTCACCATCAACGGCCT GCAGGCTGAAGATGTGGCAGTTTATTAC TGTCAGCAGTATTATAGTGAGCCCCTTTT CGGCCCTGGGACCAAAGTGGATATCAAA (SEQ ID NO: 48) | GCCCTCCAATCGGGTAACTCCCAGGAG AGTGTCACAGAGCAGGACAGCAAGGACA GCACCTACAGCCTCAGCAGCACCCTGAC GCTGAGCAAAGCAGACTACGAGAAACAC AAAGTCTACGCCTGCGAAGTCACCCAT CAGGGCCTGAGCTCGCCCGTCACAAA GAGCTTCAACAGGGGAGAGTGT (SEQ ID NO: 49) |
| RETN-5A2_LS_ 2B02 | GACATCCAGATGACCCAGTCTCCAGGC ACCCTGCTTTGCTGCAGGAGAAAGAGCC ACCCTCTCCTGCAGGGCCAGTCAGGTGTT AGGGCCAGTTACTTAGCCTGGTACCAGCA GAAACCTGGCCAGGCTCCCAGGCTCCTC ATCTATGGTACATCCAGCAGGGCCCCTG GCATCCCAGACAGGTTCAGTGGCAGTGG GTCTGGGACAGACTTCACTCTCACCATC AGCAGCCTAGAGCCTGAAGATTTTGCAG TTTATTACTGTCAGCAGCGTGGCGACTG GCTTTACACTTTTGGCCAAGGGACCAAG GTGGAGATCAAA (SEQ ID NO: 50) | AGATCTGTGGCTGCCTGCACCATCTGCT TCATCTTCCCGCCATCTGATGAGCAGTT GAAATCTGGAAACTGCCTCTGTTGTGCC TGCCTGCTGAATAAACTTCTATCCCAGAG AGGCCAAAGTACAGTGGAAGGTGATAAC GCCCTCCAATCGGGTAAACTCCCAGGAG AGTGTCACAGAGCAGGACAGCAAGGACA GCACCTACAGCCTCAGCAGCACCCTGAC GCTGAGCAAAGCAGACTACGAGAAACAC AAAGTCTACGCCCTGCGAAGTCACCCAT CAGGGCCCTGAGCTCGCCCGTCACAAA GAGCTTCAACAGGGGAGAGTGT (SEQ ID NO: 51) |
| RETN-5A2_LS_ 2D07 | CAGTCTGCCCTGACTCAGCCTGCCTCCG TGTCTGGGTCTCCTGGACAGTCGGTCAC CATCTCCTGCACTGGAACCAGCAGTGAC ATTGGTCGTTATAACCGTGTCTCCTGGTA CCAACACCACCCCGGCAAGGCCCCCAA ACTCATCATTTATGATGTCAGTAATCGGC CCTCAGGGGTCTCTAATCGCTTCTCCGG TTCCAAGTCTGGCAACACGGCCTCCCTG | AGATCTGTGGCTGCCTGCACCATCTGCT TCATCTTCCCGCCATCTGATGAGCAGTT GAAATCTGGAAACTGCCTCTGTTGTGCC TGCCTGCTGAATAAACTTCTATCCCAGAG AGGCCAAAGTACAGTGGAAGGTGATAAC GCCCTCCAATCGGGTAAACTCCCAGGAG AGTGTCACAGAGCAGGACAGCAAGGACA GCACCTACAGCCTCAGCAGCACCCTGAC |
| | ACCATCTCTGGGCTCCAGGCTGGGGAC GAGTCTGATTATTACTGCAGCGCATATAC AAGGAGCAGCACTTGGGTGTTCGGCGG AGGGACCAAGCTGACCGTCCTA (SEQ ID NO: 52) | GCTGAGCAAAGCAGCAGACTACGAGAAA CACAAAGTCTACGCCTGCGAAGTCACC CATCAGGGCCTGAGCTCGCCCGTCACA AAGAGCTTCAACAGGGGAGAGTGT (SEQ ID NO: 53) |
| RETN-5A2_LS_ 2F11 | GACATCCAGATGACCCAGTCTCCATCCT CCCTGTCTGCTTCTGTAGGAGACAGAGT CACCATCACTTGCCGGGCAAGTCAGAGC ATTGGCACCTATTTAAATTGGTATCAGCA GAAACCGGGGAAAGCTCCTAAGCTCCTG ATCTATGATGCCTCCAGTTTGGAAGATG GGGTCCCATCAAGGTTCAGCGGCAGTG GATCTGGGACAGATTTCACTCTCACCAT CAGCAGCCTGCAGCCTGAAGATTCTGCA ACTTACTACTGTCAACAGAGTTACACTAT GCCCCTTTACACTTTTGGCCAGGGGACC AAGCTGGAAATCAAA (SEQ ID NO: 54) | AGATCTGTGGCTGCCTGCACCATCTGCT TCATCTTCCCGCCATCTGATGAGCAGTT GAAATCTGGAAACTGCCTCTGTTGTGCC TGCCTGCTGAATAAACTTCTATCCCAGAG AGGCCAAAGTACAGTGGAAGGTGATAAC GCCCTCCAATCGGGTAAACTCCCAGGAG AGTGTCACAGAGCAGGACAGCAAGGACA GCACCTACAGCCTCAGCAGCACCCTGAC GCTGAGCAAAGCAGCAGACTACGAGAAA CACAAAGTCTACGCCCTGCGAAGTCACC CATCAGGGCCCTGAGCTCGCCCGTCACA AAGAGCTTCAACAGGGGAGAGTGT (SEQ ID NO: 55) |
| RETN-5A2_LS_ 2G03 | GACATCCAGATGACCCAGTCTCCATCCT CCCTGTCTGCATCTGTAGGAGACAGAGT CACCATCACTTGCCAGGCAGTCAACACG TTAGCAGCTATTTAAATTGGTATCAGC AGCAAAACCAGGGAAAGCCCCTAAGCT CCTGATCTATGCTGCATCCAATTTGCAAA GTGGGGTCCCATCAAGGTTCAGCGG CAGTGGATCTGGGACAGATTTCACTCTC ACCATCAGCAGTCTGCAACCTGATGATTT TGCAACTTATTACTGCCAACAGTATGATAG TTATTCTTCGGTGTACACCTTTGGCCC | AGATCTGTGGCTGCCTGCACCATCTGCT TCATCTTCCCGCCATCTGATGAGCAGTT GAAATCTGGAAACTGCCTCTGTTGTGCC TGCCTGCTGAATAAACTTCTATCCCAGAG AGGCCAAAGTACAGTGGAAGGTGATAAC GCCCTCCAATCGGGTAAACTCCCAGGAG AGTGTCACAGAGCAGGACAGCAAGGACA GCACCTACAGCCTCAGCAGCACCCTGAC GCTGAGCAAAGCAGCAGACTACGAGAAA CACAAAGTCTACGCCCTGCGAAGTCACC CATCAGGGCCCTGAGCTCGCCCGTCACA |
| | GGGGGACCAAGCTGGATATCAAA (SEQ ID NO: 56) | AAGAGCTTCAACAGGGGAGAGTGT (SEQ ID NO: 57) |
| RETN-5A2_LS_ 2E10 | GACATCCAGATGACCCAGTCTCCATCCT CCCTGTCTGCATCTGTAGGAGACAGAGT CACCATAACTTGCCGGACAAGTCAGACC ATAACCAACTATTTAAATTGGTATCAG CAGAAACCAGGGAGAGCCCCTAAGCTCC TGATCTTCGATGCATCCAATTTGGAAACA GGGGTCCCATCAAGATTCAGCGGCAGT GGATCTGGGACAGATTTCACTCTCACCA TCAGCAGTCTGCAACCTGAAGATTTTGC AACTTACTTCTGTCAACAGAGTTACAATC CCGGGTACACCTTTGGCCAGGGGACC AAGCTGGAGATCAAAA (SEQ ID NO: 58) | AGATCTGTGGCTGCCTGCACCATCTGCT TCATCTTCCCGCCATCTGATGAGCAGTT GAAATCTGGAAACTGCCTCTGTTGTGCC TGCCTGCTGAATAAACTTCTATCCCAGAG AGGCCAAAGTACAGTGGAAGGTGATAAC GCCCTCCAATCGGGTAAACTCCCAGGAG AGTGTCACAGAGCAGGACAGCAAGGACA GCACCTACAGCCTCAGCAGCACCCTGAC GCTGAGCAAAGCAGACTACGAGAAACAC AAAGTCTACGCCCTGCGAAGTCACCCAT CAGGGCCCTGAGCTCGCCCGTCACAAA GAGCTTCAACAGGGGGAGAGTGT (SEQ ID NO: 59) |
| RETN-5A2_LS_ 2H10 | AATTTTATGCTGACTCAGCCCGCCTCCG TGTCTGGGTCCCCTGGACAGTCGATCAC CATCTCCTGCACTGGAAGCAGCAGCGAC GTTGGTGGTTATAACTATGTCACCTGGTA CCAACAGCACCCAGGCAAAGCCCCCCAA CTCATCATTTATGATGTCACTAAGCGGCC CTCAGGGGTTTCTAATCGCTTCTCCGGC TCCAAGTCTGGCAACTCGGCCTCCCTGA CCATCTCTGGACTCCAGGCTGAGGACGA GGCTGATTATTACTGCAGCTCATACAGC AGCAGCACTTTTTACGTCTTCGGAACTG GGACCAAGGTCACCGTCCTA (SEQ ID NO: 60) | AGATCTGTGGCTGCCTGCACCATCTGCT TCATCTTCCCGCCATCTGATGAGCAGTT GAAATCTGGAAACTGCCTCTGTTGTGCC TGCCTGCTGAATAAACTTCTATCCCAGAG AGGCCAAAGTACAGTGGAAGGTGATAAC GCCCTCCAATCGGGTAAACTCCCAGGAG AGTGTCACAGAGCAGGACAGCAAGGACA GCACCTACAGCCTCAGCAGCACCCTGAC GCTGAGCAAAGCAGCAGACTACGAGAAA CACAAAGTCTACGCCTGCGAAGTCACCC ATCAGGGCCCTGAGCTCGCCCGTCACAA AGAGCTTCAACAGGGGAGAGTGT (SEQ ID NO: 61) |
| RETN-5A2 | GACATCCAGATGACCCAGTCTCCATCCT | AGATCTGTGGCTGCCTGCACCATCTGCT |
| | TCCTGTCTGCATCTGTAGGAGACAGAGT CACCATCACTTGTCGGGCCAGTCAGTA CATTGGTACTTATTTAGCCTGGTATCAGC AACAACCAGGGAAAGCCCCTAAGCTCCT GATTTATGGTGCATCCATTTTGCAAAATG GGGTCCCATCAAGGTTCAGCGGCAGTG CATCTGGGACAGAATTCACTCTCACAATC AGCAGCCTGCAGCCTGAAGATTTTGCAA CTTATTATTGTCAACAGGCTGACAGTTTC CCGCTCACTTTCGGCGGAGGGACCAAG GTGGATATCAAA (SEQ ID NO: 62) | TCATCTTCCCGCCATCTGATGAGCAGTT GAAATCTGGAACTGCCTCTGTTGTGCC TGCCTGCTGAATAACTTCTATCCCAGAG AGGCCAAAGTACAGTGGAAGGTGGATAAC GCCCTCCAATCGGGTAACTCCCAGGAG AGTGTCACAGAGCAGGACAGCAAGGACA GCACCTACAGCCTCAGCAGCACCCTGAC GCTGAGCAAAGCAGCAGACTACGAGAAA CACAAAGTCTACGCCTGCGAAGTCACCC ATCAGGGCCTGAGCTCGCCCGTCACAA AGAGCTTCAACAGGGGAGAGTGT (SEQ ID NO: 63) |

As used herein, the term "antibody" refers to an anti-resistin antibody that specifically binds to resistin, in particular human resistin. The scope of the present invention includes an antigen-binding fragment of the antibody molecule as well as a complete antibody form that specifically binds to resistin.

A complete antibody is a structure with two full-length light chains and two full-length heavy chains, and each light chain is linked to the heavy chain by a disulfide bond. The heavy chain constant region includes the gamma (γ), mu (µ), alpha (α), delta (δ), and epsilon (ε) types with subclasses gamma1 (γ1), gamma2 (γ2), gamma3 (γ3), gamma4 (γ4), alpha1 (α1), and alpha2 (α2). Constant region of light chain has kappa (κ) and lambda (λ) types.

Antigen-binding fragment or antibody fragment of an antibody refers to a fragment that possesses antigen-binding function, including Fab, F(ab'), F(ab')₂, and Fv. Among the antibody fragments, Fab has a structure with variable regions of light and heavy chains, an constant region of light chain and the first constant region (CH1) of heavy chain, and has one antigen-binding site. Fab' differs from Fab in that it has a hinge region containing one or more cysteine residues at the C-terminus of the heavy chain CH1 domain. F(ab')₂ antibody is generated by disulfide bonding of cysteine residues in the hinge region of Fab'. Recombinant techniques for generating Fv fragments with minimal antibody fragments that have only a heavy chain variable region and a light chain variable region are disclosed in PCT International Publication Nos. WO88/10649, WO88/106630, WO88/07085, WO88/07086, and WO88/09344. Two-chain Fv has a heavy chain variable region and a light chain variable region linked by non-covalent bonds, wherein a single-chain Fv (scFv) has a heavy chain variable region and a light chain variable region linked by covalent bonds, usually via a peptide linker, or directly linked at the C-terminus, which can form a dimer-like structure like a double-chain Fv. This antibody fragment can be obtained using proteolytic enzymes (e.g., restriction digestion of the whole antibody with papain yields Fab and digestion with pepsin yields F(ab')₂ fragments) or can be produced by genetic recombination techniques.

In one embodiment, the antibody according to the present invention is in the form of an Fv (e.g., scFv), or is in the form of a complete antibody. Further, the heavy chain constant region may be selected from any one of the following isotypes: gamma (γ), mu (µ), alpha (α), delta (δ), or epsilon (ε). For example, the constant region is gamma 1 (lgG1), gamma 3 (IgG3), or gamma 4 (IgG4). The light chain constant region may be the kappa or lambda isotype.

As used herein, the term "heavy chain" refers to both full-length heavy chains and fragments thereof comprising a variable region domain VH and three constant domains CH1, CH2, and CH3 comprising an amino acid sequence having sufficient variable region sequences to confer specificity to an antigen. Furthermore, as used herein, the term "light chain" refers to both full-length light chains and fragments thereof comprising a variable region domain VL and an constant region domain CL comprising an amino acid sequence having sufficient variable region sequences to confer specificity to the antigen.

The antibody of the present invention includes, but is not limited to, a monoclonal antibody, a multi-specific antibody, a human antibody, a humanized antibody, a chimeric antibody, a short-chain Fv (scFV), a short-chain antibody, a Fab fragment, a F(ab') fragment, a disulfide-bound Fv (sdFV), or an anti-idiotype (anti-ld) antibody, or an epitope-binding fragment of the antibody.

The monoclonal antibody refers to an antibody obtained from a substantially homogeneous population of antibodies, i.e., the individual antibodies in the population are identical except for possible naturally occurring mutations that may be present in trace amounts. Monoclonal antibodies are highly specific, so they are directed against a single antigenic site. In contrast to conventional (polyclonal) antibody preparations, which typically contain different antibodies directed against different determinants (epitopes), each monoclonal antibody is directed against a single determinant on an antigen.

For example, a monoclonal antibody useful in the present invention can be produced by hybridoma methods, or can be produced using recombinant DNA methods in bacterial, eukaryotic, or plant cells (see U.S. Patent No. 4,816,567). Additionally, monoclonal antibodies can be isolated from phage antibody libraries.

An "epitope" is a protein determinant to which an antibody can specifically bind. An epitope typically consists of a group of chemically active surface molecules, such as amino acids or sugar side chains, which typically have specific charge characteristics as well as specific three-dimensional structural features. Steric and amorphous epitopes are distinguished in that binding to the former is lost in the presence of a denaturing solvent, but not to the latter.

Non-human (e.g., murine) antibodies in their "humanized" form are chimeric antibodies containing a minimal sequence derived from a non-human immunoglobulin. In most cases, a humanized antibody is a human immunoglobulin (recipient antibody) wherein residues from the hypervariable region of the recipient are replaced with residues from the hypervariable region of a non-human species (donor antibody), such as mouse, rat, rabbit, or non-human primate, that possesses the specificity, affinity, and ability to target the recipient.

The "human antibody" means a molecule derived from a human immunoglobulin, wherein the entire amino acid sequence comprising the antibody, including complementary crystal regions and structural regions, is composed of human immunoglobulin.

It includes not only a "chimeric" antibody (immunoglobulin) in which a portion of the heavy and/or light chain is identical or homologous to a corresponding sequence in an antibody derived from a particular species or belonging to a particular antibody class or subclass, while the remaining chain(s) are identical or homologous to a corresponding sequence in an antibody derived from another species or belonging to another antibody class or subclass, but also fragments of the antibodies exhibiting the intended biological activity.

As used herein, "antibody variable domain" refers to the light and heavy chain portions of an antibody molecule that comprises the amino acid sequences of the complementarity determining region (CDR; i.e., CDR1, CDR2, and CDR3), and the backbone region (FR). VH refers to the variable domain of the heavy chain. VL refers to the variable domain of the light chain.

The "complementarity determining region" (CDR; i.e., CDR1, CDR2, and CDR3) refers to the amino acid residue in an antibody variable domain that is required for antigen binding. Each variable domain typically has three CDR regions, identified as CDR1, CDR2, and CDR3.

"Fv" fragment is an antibody fragment that contains a complete antibody recognition and binding sites. This region consists of one heavy chain variable domain and one light chain variable domain tightly and virtually covalently associated as a dimer, e.g., scFv.

"Fab" fragment contains variable and constant domains in the light chain, and variable and first constant domains (CH1) in the heavy chain. F(ab')₂ antibody fragments typically contain a pair of Fab fragments that are covalently linked near their carboxy terminus by a hinge cysteine between them.

A "single-chain Fv" or "scFv" antibody fragment contains the VH and VL domains of the antibody, which are present within a single polypeptide chain. The Fv polypeptide may additionally contain a polypeptide linker between the VH and VL domains that allows the scFv to form the targeted structure for antigen binding.

The antibody according to the present invention is an antibody with increased affinity for an antigen. The term "affinity" refers to the ability to specifically recognize and bind to a specific site on an antigen, and a high degree of affinity, is an important factor in the immune response, together with the specificity of the antibody to the antigen. Affinity can be determined using any of a variety of assays known in the art, such as radioimmunoassay (RIA) and ELISA, and can be expressed with a variety of quantitative values. The affinity of an antibody to an antigen can generally be expressed by the dissociation constant (Kd) of a particular antibody-antigen interaction. The lower the value of Kd, the higher the affinity of the antibody for the antigen.

The antibody or antibody fragments of the present invention can comprise not only the sequences of the anti-resistin antibody of the present invention described herein, but also biological equivalents thereof, to the extent that they are capable of specifically recognizing resistin. For example, additional modifications can be made to the amino acid sequence of the antibody to further improve the binding affinity and/or other biological properties of the antibody. Such modifications include, for example, deletions, insertions, and/or substitutions of amino acid sequence residues of the antibody. These amino acid modifications are made based on the relative similarity of the amino acid side chain substituents, e.g., hydrophobicity, hydrophilicity, charge, size, etc. By analyzing the size, shape, and type of amino acid side chain substituents, it can be seen that arginine, lysine, and histidine are all positively charged residues; alanine, glycine, and serine have similar sizes; and phenylalanine, tryptophan, and tyrosine have similar shapes. Therefore, based on these considerations, arginine, lysine, and histidine; alanine, glycine, and serine; and phenylalanine, tryptophan, and tyrosine are biologically functional equivalents.

When considering the variants with biologically equivalent activity described above, the antibody of the present invention or the nucleic acid molecule encoding the same is also interpreted to include sequences that exhibit substantial identity to the sequences set forth in the sequence ID numbers. This substantial identity means a sequence that exhibits at least 90% homology, most preferably at least 95% homology, at least 96% homology, at least 97% homology, at least 98% homology, at least 99% homology, when the sequence of the present invention is aligned with any other sequence as closely as possible, and the aligned sequence is analyzed using algorithms known in the art. Alignment methods for sequence comparison are known in the art. The NCBI basic local alignment search tool (BLAST) is accessible at NBCI and elsewhere, and is available on the Internet in conjunction with sequence analysis programs such as blastp, blasm, blastx, tblastn, and tblastx. BLSAT can be accessed at www.ncbi.nlm.nih.gov/BLAST/. Methods for sequence homology comparison using this program are available at www.ncbi.nlm.nih.gov/BLAST/blast_ help.html.

Based on this, an antibody or antigen-binding fragment thereof of the present invention may have 90%, 91%, 92%, 93%, 94%, 95%, 96%, 97%, 98%, 99%, or greater homology compared to the specified or whole sequences set forth in the specification. Such homology can be determined by sequence comparison and/or alignment by methods known in the art. For example, sequence comparison algorithms (e.g., BLAST or BLAST 2.0), manual alignment, and visual inspection can be used to determine the percent sequence homology of the nucleic acids or proteins of the present invention.

In another aspect, the present invention relates to a nucleic acid encoding a heavy chain variable region of the antibody or antigen-binding fragment thereof.

In the present invention, it is preferably a nucleic acid encoding a heavy chain variable region of an antibody that binds specifically to resistin comprising the amino acid sequence of SEQ ID NO: 28, and more preferably a nucleic acid encoding the amino acid sequence of SEQ ID NO: 30, and the sequences are shown in Table 4 above.

In still another aspect, the present invention relates to a nucleic acid encoding a light chain variable region of the antibody or antigen-binding fragment thereof.

In the present invention, it is preferably a nucleic acid encoding a light chain variable region of an antibody that binds specifically to resistin comprising an amino acid sequence selected from the group consisting of SEQ ID NOs: 32, 34, 36, 38, 40, 42, 44 and 46, and more preferably a nucleic acid selected from the group consisting of SEQ ID NOs: 48, 50, 52, 54, 56, 58, 60 and 62, and the sequences are shown in Table 6 above.

The term "nucleic acid" encompasses DNA (gDNA and cDNA) and RNA molecules, and the nucleotide, the basic unit of a nucleic acid, includes not only naturally occurring nucleotides, but also analogs with sugar or base site modifications. The sequences of the nucleic acid encoding the heavy and light chain variable regions of the present invention may be modified. The modifications include additions, deletions, or non-conservative or conservative substitutions of nucleotides.

The DNA encoding the antibody is readily isolated or synthesized using conventional procedures (e.g., by using oligonucleotide probes that can specifically bind to DNA encoding the heavy and light chains of the antibody). Many vectors are available. Vector components typically include, but are not limited to, one or more of the following: a signal sequence, an origin of replication, one or more marker genes, an enhancer element, a promoter, and a transcription termination sequence.

An antibody or antigen-binding fragment thereof can be produced recombinantly by isolating the nucleic acid encoding the antibody or antigen-binding fragment thereof of the present invention. The nucleic acid is isolated, and inserted into a replicable vector for further cloning (amplification of DNA) or further expression.

Based on this, in yet another aspect, the present invention relates to a vector comprising the nucleic acid.

As used herein, the term "vector" includes a plasmid vector; a cosmid vector; and a viral vector such as a bacteriophage vector, an adenoviral vector, a retroviral vector, and an adeno-associated viral vector as a means for expressing a target gene in a host cell. In the vectors, the nucleic acid encoding the antibody is operatively linked to a promoter.

"Operationally linked" means a functional association between a nucleic acid expression regulatory sequence (e.g., a promoter, signal sequence, or array of transcriptional regulator binding sites) and another nucleic acid sequence, whereby the regulatory sequence regulates the transcription and/or decoding of the other nucleic acid sequence.

In the case of prokaryotic cells as hosts, it is common to include a strong promoter capable of driving transcription (e.g., tac promoter, lac promoter, lacUV5 promoter, Ipp promoter, pLλ promoter, pRλ promoter, rac5 promoter, amp promoter, recA promoter, SP6 promoter, trp promoter, and T7 promoter), a ribosome binding site for initiation of degradation, and a transcription/degradation termination sequence. In addition, for example, in the case of eukaryotic cells as hosts, a promoter derived from the genome of a mammalian cell (e.g., metallothionein promoter, β-actin promoter, human hemoglobin promoter, and human muscle creatine promoter) or a promoter derived from a mammalian virus (e.g., adenovirus late promoter, vaccinia virus 7.5K promoter, SV40 promoter, cytomegalovirus (CMV) promoter, tk promoter of HSV, mouse mammary tumor virus (MMTV) promoter, LTR promoter of HIV, promoter of Moloney virus, promoter of Epstein-Barr virus (EBV), and promoter of Roos Sakoma virus (RSV)) may be utilized and typically have a polyadenylation sequence as a transcription termination sequence.

In some cases, the vector may be fused with other sequences to facilitate purification of antibodies expressed therefrom. Examples of fused sequences include glutathione S-transferase (Pharmacia, USA), maltose binding protein (NEB, USA), FLAG (IBl, USA), and 6x His (hexahistidine; Quiagen, USA).

The vector comprises as selective markers antibiotic resistance genes conventionally used in the art, for example, resistance genes to ampicillin, gentamicin, carbenicillin, chloramphenicol, streptomycin, kanamycin, geneticin, neomycin, and tetracycline.

The vector expressing the above resistin-specific antibody or antigen-binding fragment thereof can be either a vector system in which the nucleic acid encoding the heavy chain variable region and the nucleic acid encoding the light chain variable region are simultaneously expressed in one vector, or a system in which the light and heavy chains are expressed in separate vectors. In the latter case, the two vectors can be introduced into the host cell by co-transfection and targeted transformation. Furthermore, cells transformed with a vector comprising a light chain (or heavy chain) can be screened and the selected cells can be transformed again with a vector comprising a heavy chain (or light chain) to finally select for cells expressing both light and heavy chains.

Preferably, the vector system is one in which a nucleic acid encoding a heavy chain variable region of a resistin-specific antibody or antigen-binding fragment thereof and a nucleic acid encoding a light chain variable region are simultaneously expressed in one vector, but not limited thereto.

In further another aspect, the present invention relates to cells transformed with the above-mentioned vectors.

In the present invention, the cells may be selected from the group consisting of, but not limited to, animal cells, plant cells, yeast, *E. coli,* and insect cells.

The cells used to generate the antibody of the present invention can be, but are not limited to, prokaryotic, yeast, or higher eukaryotic cells.

Prokaryotic host cells such as *Escherichia coli,* strains of the Bacillus genus, such as Bacillus subtilis and Bacillus churrigensis, Streptomyces, Pseudomonas (e.g, *Pseudomonas putida*), *Proteus mirabilis,* and Staphylococcus (e.g., *Staphylococcus carnosus)* can be used.

However, animal cells are of greatest interest, and examples of useful host cell lines include, but are not limited to, BHK, CHO, CHOK1, DXB-11, DG-44, CHO/-DHFR, CV1, COS-7, HEK293, TM4, VERO, HELA, MDCK, BRL 3A, W138, Hep G2, SK-Hep, MMT, TRI, MRC 5, FS4, 3T3, RIN, A549, PC12, K562, PER.C6, SP2/0, NS-0, U20S, or HT1080.

In still yet another aspect, the present invention relates to a method of producing an antibody or antigen-binding fragment thereof, comprising (a) culturing the cells; and (b) recovering the antibody or antigen-binding fragment thereof from the cultured cells.

The cells can be cultured in a variety of media. Any of the commercially available media can be used as culture media without limitation. All other necessary supplements known to those skilled in the art may also be included in suitable concentrations. Culture conditions, e.g., temperature, pH, etc., are already used with host cells selected for expression and would be apparent to those skilled in the art.

Recovery of the antibody or antigen-binding fragment thereof can be accomplished by removing impurities, for example by centrifugation or ultrafiltration, and purifying the resulting product, for example by affinity chromatography. Additional other purification techniques may be used, for example, anion or cation exchange chromatography, hydrophobic interaction chromatography, or hydroxylapatite chromatography.

In still further another aspect, the present invention relates to an antibody-drug conjugate (ADC) in which a drug is conjugated to the antibody that specifically binds to resistin or antigen-binding fragment thereof.

Antibody-drug conjugate requires the drug to be stably bound to the antibody before the drug can be delivered to the target cell. Once delivered to the target, the drug, for example, an anticancer drug, must be separated from the antibody and induce the death of the target cell. This requires the drug to be stably bound to the antibody and at the same time have sufficient cytotoxicity to induce the death of the target cell when it is separated from the antibody.

In the present invention, the antibody or antigen-binding fragment thereof and a cytotoxic substance, including a drug such as an anticancer agent, may be linked to each other (e.g., by covalent bonds, peptide bonds, etc.) and used in the form of a conjugate or fusion protein (in case that the cytotoxic substance and/or the labeled substance is a protein). The cytotoxic agent may be any substance that is toxic to cancer cells, in particular solid cancer cells, and may be one or more selected from the group consisting of, but not limited to, radioisotopes, cytotoxic compounds (small molecules), cytotoxic proteins, and anticancer drugs. The cytotoxin protein may be one or more selected from the group consisting of, but not limited to, ricin, saporin, gelonin, momordin, debouganin, diphtheria toxin, and pseudomonas toxin. The radioisotope may be one or more selected from the group consisting of, but not limited to, 131I, 188Rh, and 90Y. The cytotoxin compound may be at least one selected from the group consisting of, but not limited to, duocarmycin, monomethyl auristatin E (MMAE), monomethyl auristatin F (MMAF), N2'-deacetyl-N2'-(3-mercapto-1-oxopropyl)maytansine (DM1), and pyrrolobenzodiazepine (PBD) dimer.

In the present invention, the antibody-drug conjugate may be according to techniques well known in the art.

In the present invention, the antibody-drug conjugate may be characterized in that the antibody or antigen-binding fragment thereof are linked to a drug via a linker.

In the present invention, the linker may be a cleavable linker or a non-cleavable linker.

The linker is a linking site between the anti-resistin antibody and the drug, for example, the linker is in a cleavable form under intracellular conditions, such that the drug can be released from the antibody via cleavage of the linker in an intracellular environment.

The linker can be cleaved by cleavage agents present in the intracellular environment, for example in lysosomes or endosomes, and can be a peptide linker that can be cleaved by intracellular peptidase or protease enzymes, for example lysosomal or endosomal proteases. Typically, a peptide linker has a length of at least two amino acids. The cleavage agent may include cathepsin B and cathepsin D, and plasmin, and may hydrolyze the peptide to release the drug into the target cell. The peptide linker may be cleaved by the thiol-dependent protease cathepsin-B, which is highly expressed in cancer tissue, and for example, a Phe-Leu or Gly-Phe-Leu-Gly linker may be used. Further, the peptide linker may be cleaved by an intracellular protease, for example, and may be a Val-Cit linker or a Phe-Lys linker.

In the present invention, the cleavable linker may be pH sensitive, meaning that it is sensitive to hydrolysis at certain pH values. In general, a pH-sensitive linker indicates that it can be hydrolyzed under acidic conditions. For example, acid-labile linkers that can be hydrolyzed in lysosomes may be, for example, hydrazones, semicarbazones, thiosemicarbazones, cis-aconitic amides, orthoesters, acetals, or ketals.

The linker may also be cleaved under reducing conditions, for example, a disulfide linker. Various disulfide bonds can be formed using SATA (N-succinimidyl-S-acetylthioacetate), SPDP (N-succinimidyl-3-(2-pyridyldithio)propionate), SPDB (N-succinimidyl-3-(2-pyridyldithio)butyrate), and SMPT (N-succinimidyl-oxycarbonyl-alphamethyl-alpha-(2-pyridyl-dithio)toluene).

In the present invention, the drug and/or drug-linker may be conjugated randomly via lysines of the antibody or via cysteines that are exposed when the disulfide bond chain is reduced. In some cases, the linker-drug may be conjugated via a genetically engineered tag, such as cysteine present in a peptide or protein. The genetically engineered tag, e.g., peptide or protein, may comprise, for example, an amino acid motif that can be recognized by an isoprenoid transferase. The peptide or protein has a deletion at the carboxy terminus of the peptide or protein, or has an addition via covalent bond of a spacer unit to the carboxy (C) terminus of the peptide or protein. The peptide or protein may be covalently linked directly to the amino acid motif, or covalently linked to the spacer unit and linked to the amino acid motif. The amino acid spacer unit consists of 1 to 20 amino acids, preferably glycine units.

The linker may comprise a beta-glucuronide linker that is recognized and hydrolyzed by β-glucuronidase, which is abundant in lysosomes or overexpressed in some tumor cells. Unlike peptide linkers, it has the advantage of being highly hydrophilic, which can increase the solubility of the antibody-drug complex when combined with drugs that are more hydrophobic.

In this regard, beta-glucuronide linkers disclosed in Korean Laid-open Patent Publication No. 2015-0137015, for example, beta-glucuronide linkers comprising self-immolative groups, can be used in the present invention.

In addition, the linker may be, for example, a non-cleavable linker, wherein the drug is released after only one step of antibody hydrolysis to produce, for example, an amino acid-linker-drug complex. This type of linker may be a thioether or maleimidocaproyl group, and may be stable in blood.

In the present invention, the drug may be a chemotherapeutic agent, toxin, micro RNA (miRNA), siRNA, shRNA, or radioisotope. The drug may be conjugated to an antibody in a formulation that exhibits a pharmacologic effect.

The chemotherapeutic agent may be a cytotoxic agent or an immunosuppressive agent. Specifically, it may include a chemotherapeutic agent that can function as a microtubulin inhibitor, a mitotic inhibitor, a topoisomerase inhibitor, or a DNA intercalator. It can also include an immunomodulatory compound, an anticancer agent, an antiviral agent, an antibacterial agent, an antifungal agent, an anthelmintic agent, or a combination thereof.

The drug may be at least one selected from the group consisting of mytansinoid, orlistatine, aminopterin, actinomycin, bleomycin, thalidomide, camptothecin, N8-acetyl spermidine, 1-(2-chloroethyl)-1,2-dimethylsulfonyl hydrazide, esperamycin, etoposide, 6-mercaptopurine, dolastatin, trichothecene, kalikeamycin, taxol, taxane, paclitaxel, docetaxel, methotrexate, vincristine, vinblastine, doxorubicin, melphalan, chlorambucil, duocamycin, L-asparaginase, mercaptopurine, thioguanine, hydroxyurea, cytarabine, cyclophosphamide, ifosfamide, nitrosourea, cisplatin, carboplatin, mitomycin (mitomycin A, mitomycin C), dacarbazine, procarbazine, topotecan, nitrogen mustard, cytoxan, 5-fluorouracil, CNU (bischloroethylnitrosourea), irinotecan, camptothecin, idarubicin, daunorubicin, dactinomycin, plicamycin, asparaginase, vinorelbine, chlorambucil, melphalan, carmustine, lomustine, busulfan, treosulfan, decarbazine, teniposide, topotecan, 9-aminocamptothecin, crisnatol, trimetrexate, mycophenolic acid, tiazofurin, ribavirin, EICAR (5-ethynyl-1-beta-Dribofuranosylimidazole-4-carboxamide), hydroxyurea, deferoxamine, floxuridine, doxifluridine, raltitrexed, cytarabine (ara Cand cytosine arabinoside, fludarabine, tamoxifen, raloxifene, megestrol, goserelin, leuprolide acetate, flutamide, bicalutamide, EB1089, CB1093, KH1060, verteporfin, phthalocyanine, photosensitizer Pe4, demethoxy-hypocrellin A, interferon-α, interferon-γ, tumor necrosis factor, gemcitabine, velcade, Revlimid, lovastatin, 1-methyl-4-phenylpyridinium ion, staurosporine, actinomycin D, dactinomycin, bleomycin A2, bleomycin B2, peplomycin, epirubicin, pirarubicin, zorubicin, mitoxantrone, verapamil, thapsigargin, nucleases, and toxins of bacterial or animal origin, but not limited thereto.

In the present invention, the drug may comprise one or more nucleophiles selected from the group consisting of amine, thiol, hydroxyl, hydrazide, oxime, hydrazine, thiosemicarbazone, hydrazine carboxylate, and arylhydrazide groups that are reactive to form covalent bonds with electrophilic groups on the linker and linker reagent.

In yet further another aspect, the present invention relates to a bispecific or multi-specific antibody comprising the antibody that specifically binds to resistin or antigen-binding fragment thereof.

A bispecific antibody is an antibody that binds or antagonizes more than one target, or a combination of antibodies that bind or antagonize two different targets, or an antibody in which an antibody that binds to one target and a substance that antagonizes another target.

A multi-specific antibody is an antibody that has binding specificity for at least three different antigens. A multi-specific antibody may comprise an antibody that is more than tri-specific, such as a tri-specific antibody, a tetra-specific antibody, or an antibody that targets more targets.

Methods for the production of bispecific or multi-specific antibodies are widely known in the art. Traditionally, the recombinant production of bispecific antibodies is based on the co-expression of two or more immunoglobulin heavy/light chain pairs under conditions where the two or more heavy chains have different specificities.

The antigen to which the antibody, other than an anti-resistin antibody comprised in the bispecific or multi-specific antibody, binds is preferably a cancer-associated antigen or immune checkpoint protein antigen, which can be selected from the group consisting of HGF, EGFR, EGFRvlll, Her2, Her3, IGF-1R, VEGF, VEGFR-1, VEGFR-2, VEGFR-3, Ang2, DII4, NRP1, FGFR, FGFR2, FGFR3, c-Kit, MUC1, MUC16, CD20, CD22, CD27, CD30, CD33, CD40, CD52, CD70, CD79, DDL3, Folate R1, Nectin 4, Trop2, gpNMB, Axl, BCMA, PD-1, PD-L1, PD-L2, CTLA4, BTLA, 4-1BB, ICOS, GITR, OX40, VISTA, TIM-3, LAG-3, KIR, B7.1, B7.2, B7-H2, B7-H3, B7-H4, B7-H6, B7-H7, EphA2, EphA4, EphB2, E-selectin, EpCam, CEA, PSMA, PSA, and c-MET, and an immunocompetent cell-associated antigen may be selected from TCR/CD3, CD16 (FcγRIIIa) CD44, CD56, CD69, CD64 (FcγRI), CD89, or CD11b/CD18 (CR3), but not limited thereto.

In another aspect, the present invention relates to a chimeric antigen receptor (CAR) comprising the antibody that specifically binds to resistin or antigen-binding fragment thereof.

A CAR can comprise an antigen binding domain, a transmembrane domain, and an intracellular signaling domain, wherein the antigen binding domain can be linked to the transmembrane domain by a linker. The extracellular domain comprising the antigen binding domain may also comprise a signal peptide.

In still another aspect, the present invention relates to an immune cell comprising the chimeric antigen receptor.

The immune cells may comprise cells genetically modified to express the chimeric antigen receptor, preferably be in that they are T cells or NK cells.

In yet another aspect, the present invention relates to a pharmaceutical composition for the prevention or treatment of a disease that can be treated by inhibiting the activity of risistin comprising the antibody or antigen-binding fragment thereof, the antibody-drug conjugate, the bi- or multi-specific antibody, or the chimeric antigen receptor.

In the present invention, the activity of resistin may be inhibited by blocking the resistin/CAP1 binding by the resistin antibody.

In the present invention, the diseases that can be treated by inhibiting the activity of resistin are preferably cancer, cardiovascular metabolic diseases, autoimmune diseases, or inflammatory diseases, but not limited thereto.

The invention may be, for example, a pharmaceutical composition for the prevention or treatment of cancer, cardiovascular metabolic disease, autoimmune disease, or inflammatory disease, comprising (a) a pharmaceutically effective amount of the antibody or antigen-binding fragment thereof that specifically bind to resistin according to the present invention; and (b) a pharmaceutically acceptable carrier. The present invention also relates to a method of preventing or treating cancer, cardiovascular metabolic disease, autoimmune disease, or inflammatory disease, comprising administering to a patient the antibody or antigen-binding fragment thereof that specifically binds to resistin according to the present invention, in an effective amount required for the patient.

Accordingly, in further another aspect, the present invention relates to a method of preventing or treating a disease that can be treated by inhibiting the activity of resistin, comprising administering to a subject a composition comprising the antibody or antigen-binding fragment thereof, the antibody-drug conjugate, the bi- or multi-specific antibody, or the chimeric antigen receptor.

In still yet another aspect, the present invention relates to the use of the antibody or antigen-binding fragment thereof, the antibody-drug conjugate, the bi- or multi-specific antibody or the chimeric antigen receptor, or the composition comprising the same, for the prevention or treatment of a disease that can be treated by inhibition of the activity of resistin.

In still further another aspect, the present invention relates to the use of the antibody or antigen-binding fragment thereof, the antibody-drug conjugate, the bi- or multi-specific antibody or the chimeric antigen receptor, or the composition comprising the same, in the manufacture of a medicament for preventing or treating a disease that can be treated by inhibition of the activity of resistin.

Since the composition, method, usage and use utilize the anti-resistin antibody of the present invention or antigen-binding fragment thereof as active ingredients, duplicate descriptions are omitted.

"Prevention" means any act of inhibiting or delaying the progression of a disease that can be treated by the administration of a composition according to the present invention, resulting in inhibition of the activity of resistin, and "treatment" means inhibition of the development of a disease that can be treated by inhibition of the activity of resistin, or mitigation or elimination of the disease.

The terms "cancer" and "cancerous" refer to or describe a physiological condition in mammals in which a population of cells undergoes uncontrolled cell growth.

A "tumor" is any mass of tissue caused by excessive cell growth or proliferation that is either benign (non-cancerous) or malignant (cancerous), including pre-cancerous lesions.

The terms "cancer cells," "tumor cells," and their grammatical equivalents refer to the total population of cells derived from a tumor or precancerous lesion, including both non-tumorigenic cells, which comprise the bulk of the tumor cell population, and tumorigenic stem cells (cancer stem cells).

In the present invention,"cancer" includes, without limitation, any cancer in which resistin is expressed. The resistin-expressing cancer is preferably selected from the group consisting of, but not limited to, breast cancer, metastatic cancer, uterine cancer, ovarian cancer, prostate cancer, melanoma, lung cancer, liver cancer, glioblastoma, colon cancer, head and neck cancer, bladder cancer, renal cell carcinoma, gastric cancer, pancreatic cancer, and recurrent cancer.

In particular, an antibody or antigen-binding fragment thereof or a pharmaceutical composition comprising the same of the present invention may inhibit the metastasis of cancer.

In the present invention, "cardiovascular metabolic disease, autoimmune disease, or inflammatory disease" includes, without limitation, any disease in which resistin is expressed.

In the present invention, the cardiovascular metabolic disease is preferably selected from the group consisting of obesity, hyperlipidemia, hypertension, atherosclerosis, hyperinsulinemia, insulin resistant diabetes, type 2 diabetes, liver disease, non-alcoholic fatty liver disease (NAFLD) and non-alcoholic steatohepatitis (NASH), but not limited thereto.

In the present invention, the autoimmune disease is preferably selected from the group consisting of chronic heart disease, rheumatoid arthritis, systemic lupus erythematosus, digestive diabetes, atopic dermatitis, autoimmune encephalomyelitis, asthma, and Crohn's disease, but not limited thereto.

In the present invention, the inflammatory disease may be preferably selected from the group consisting of inflammatory skin diseases such as asthma, eczema, psoriasis, acne, allergies, rheumatoid arthritis, psoriatic arthritis, atopic dermatitis, atopic rhinitis, allergic dermatitis, chronic sinusitis, or seborrheic dermatitis; inflammatory bowel disease (IBD) such as Crohn's disease or ulcerative colitis; and acute or chronic inflammatory diseases such as ankylosing spondylitis, sepsis, septic shock, vasculitis, and bursitis, but not limited thereto.

The pharmaceutical composition of the present invention may comprise an antibody specific for resistin or fragments thereof, and may further comprise a pharmaceutically acceptable carrier for administration of the pharmaceutical composition of the present invention.

As used herein, the term "pharmaceutically acceptable carrier" refers to a carrier or diluent that does not irritate the organism and does not inhibit the biological activity and properties of the administered compound. Pharmaceutically acceptable carriers for compositions formulated as liquid solutions may be sterile and biocompatible, and may include saline, sterile water, buffered saline, albumin injectable solution, dextrose solution, maltodextrin solution, glycerol, and mixtures of one or more of these components, and other conventional additives such as antioxidants, buffers, or bacteriostatic agents, as needed. It can also be formulated into injectable formulations such as aqueous solutions, suspensions, or emulsions, pills, capsules, granules, or tablets with additional diluents, dispersants, surfactants, binders, or lubricants.

The pharmaceutical composition of the present invention can be in various different formulations, either oral or parenteral. When formulated, they are usually prepared using diluents or excipients such as fillers, bulking agents, binders, wetting agents, disintegrating agents, or surfactants. Solid dosage forms for oral administration include tablets, pills, powders, granules, capsules, etc. These solid dosage forms are prepared by mixing one or more compounds with at least one excipient, such as starch, calcium carbonate, sucrose or lactose, gelatin, etc. In addition to simple excipients, lubricants such as magnesium stearate, or talc are also used. Liquid preparations for oral administration include suspensions, solutions, emulsions, and syrups, and may comprise a variety of excipients, such as wetting agents, sweeteners, flavors, or preservatives, in addition to the commonly used simple diluents such as water or liquid paraffin. Formulations for parenteral administration include sterile aqueous solutions, non-aqueous solutions, suspensions, emulsions, lyophilizates, and suppositories. Non-aqueous solvents and suspensions may include propylene glycol, polyethylene glycol, vegetable oils such as olive oil, and injectable esters such as ethylolate. As a base for the suppository, witepsol, macrogol, tween 61, cacao oil, laurin oil, or glycerogelatin can be used.

In yet further another aspect, the present invention provides a method of inhibiting the growth, migration, or metastasis of a resistin-expressing tumor cell, comprising contacting the antibody or antigen-binding fragment thereof.

The tumor cells include, without limitation, any type of tumor cell that expresses resistin. Preferably, the resistin-expressing tumor cells may be, but are not limited to, breast cancer, metastatic cancer, uterine cancer, ovarian cancer, prostate cancer, melanoma, lung cancer, liver cancer, glioblastoma, colon cancer, head and neck cancer, bladder cancer, renal cell carcinoma, gastric cancer, pancreatic cancer, or recurrent cancer cells.

The present invention relates to a method of treating a cancer, cardiovascular metabolic disease, autoimmune disease, or inflammatory disease, comprising the step of administering to an individual a pharmaceutically effective amount of an antibody or antigen-binding fragment thereof.

The method of treatment with the antibody or antigen-binding fragment thereof of the present invention comprises administering the antibody or antigen-binding fragment thereof in a pharmaceutically effective amount. It would be apparent to those skilled in the art that a suitable total daily dose may be determined by the treating physician within the scope of sound medical judgment and may be administered in a single or multiple doses. However, for the purposes of the present invention, the specific therapeutically effective amount for a particular patient would preferably vary depending on a variety of factors such as the type and degree of response to be achieved, a specific composition including whether other preparations are used in some cases, the patient's age, weight, general health, sex and diet, the time of administration, route of administration and duration of treatment, drugs used in combination with or concurrently with the specific composition, and similar factors well known in the field of medicine.

An individual to whom the composition of the present invention is administered includes, without limitation, a mammal, including a human.

The term "administer" in the present invention refers to the introduction of a pharmaceutical composition of the present invention into a patient by any suitable means, and the route of administration of a composition of the present invention may be via a variety of routes, oral or parenteral, as long as it can reach the target tissue.

Hereinafter, the present invention will be described in more detail with reference to examples. However, it will be obvious to those skilled in the art that these examples are provided only for illustration of the present invention, and should not be construed as limiting the scope of the present invention.

### Example 1: Expression and purification of resistin antigen

To express the RETN-C-Fc protein, in which a human antibody Fc sequence is fused to the C-terminus of the resistin sequence, and the RETN-flag-His protein, in which a flag sequence and a histidine sequence are fused to the C-terminus of the resistin sequence, in animal cells, HEK-293F cells were transfected with RETN-flag-His DNA, and incubated for a period of time, and the cultures were harvested. For transfection, DNA was added to the Freestyle 293 expression culture and 200 µg of polyethylenimine (PEI) was added to make a polyplex reaction. The polyplex reaction was incubated for 20 min at room temperature, then added to 1×10⁶ cells/80 ml of HEK293F cell culture flask incubated in HEK293 culture medium, and then incubated for 7 days at 37°C, CO₂ 8%, 85 rpm.

After 7 days, the cultures were harvested and centrifuged at 5000 rpm for 10 min to precipitate and remove cells and cellular debris in the culture. To purify and obtain the antigenic protein in the obtained supernatant, the supernatant of the RETN-C-Fc antigen DNA transfection culture was reacted with a column loaded with recombinant protein A agarose resin at 4°C for 16 hours to adsorb the antigenic protein to the resin, and then eluted with 0.1 M pH3.3 glycine solution. The eluted proteins were neutralized to pH 7-7.4 with 1 M Tris-HCl solution, and the solvent was replaced with DPBS using Maxi GeBAflex-tubes (12000-14000MWCO, D050-100). To purify the RETN-flag-His antigen, the culture supernatant was reacted with a column loaded with Ni Sepharose 6Fast Flow (GE healthcare, 17531803) resin for 16 hours at 4°C to adsorb the antigenic proteins to the resin, followed by elution with 500 mM imidazole solution. The eluted proteins were solvent exchanged with DPBS and concentrated using Maxi GeBAflex-tubes (12000-14000MWCO, D050-100).

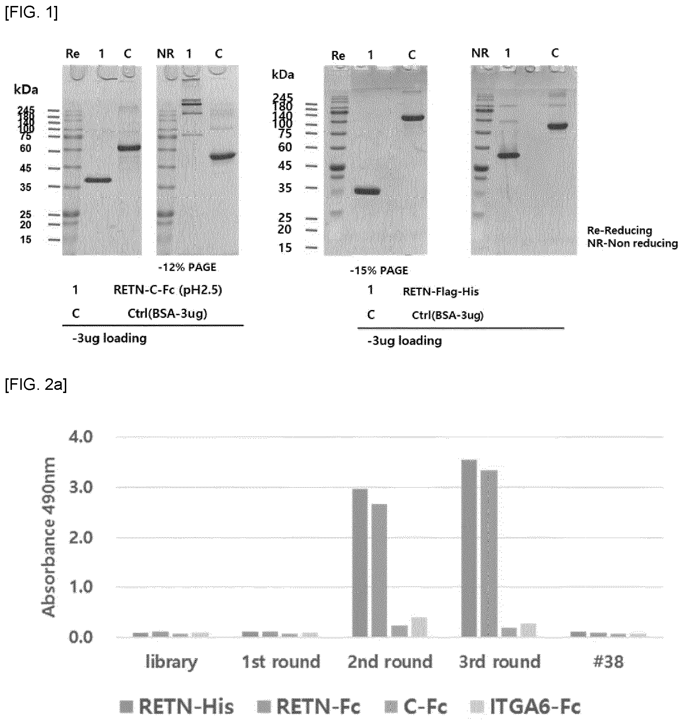

The purified proteins were determined for purity using SDS-PAGE gels (FIG. 1).

### Example 2: Primary screening of resistin human antibody

### Example 2-1: Biopanning

To obtain a pool of phages that bind to a resistin antigen, biopanning was performed. RETN-C-Fc or RETN-flag-His antigens produced in Example 1 were coated onto immunosorb tubes and blocked. Human antibody library phage was prepared by infecting human scFv libraries with *E. coli* and culturing them, and then harvesting and concentrating the cultures. The human antibody library phage was incubated in antigen-coated immunotubes, and the scFv phage bound to the antigen was eluted. The phage obtained from the first round of panning was recovered, re-infected with *E. coli,* amplified, and subjected to second and third rounds of biopanning using RETN-C-Fc or RETN-flag-His antigens. A pool of polyphages binding to the antigen was obtained in each round.

### Example 2-2: Polyphage ELISA

A polyphage ELISA was performed to determine the degree of specific binding to antigens of the scFv phage pool obtained in each round. As specific antigens, RETN-flag-His antigen and RETN-C-Fc antigen were coated at 100 ng/well in 96-well immune plates (NUNC, 439454) and blocked with skim milk. Each well was treated and reacted with the scFv phage obtained in each round, washed with PBS-T, and reacted with anti-M13-HRP (Amersham, 27-9421-01) as a secondary antibody at 1:2000. After PBS-T washing, 100 µl of OPD (sigma, 8787-TAB) solution was applied to each well for 10 min of substrate reaction. After 10 minutes, the substrate reaction was stopped by treating each well with 50 µl of 1N H₂SO₄ and measured at 490 nm using a spectrophotometer (thermo fisher scientific, 51119200).

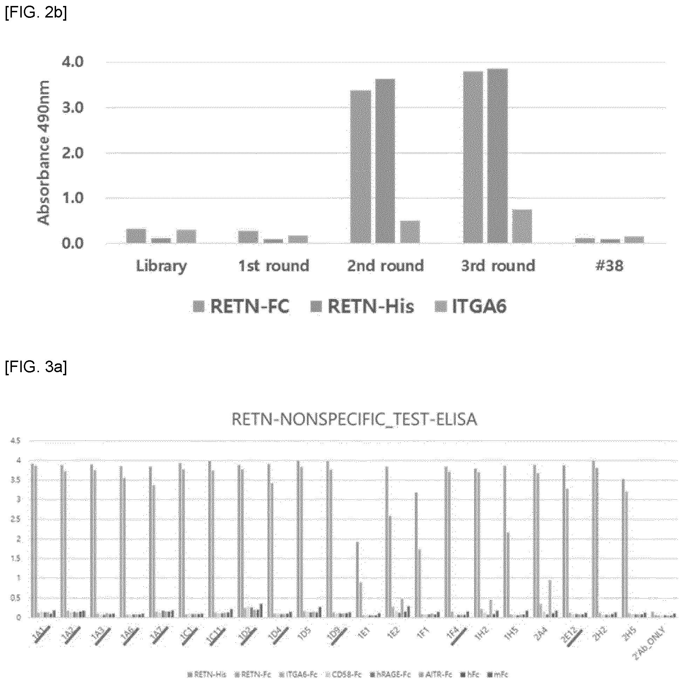

The results showed that the antigen-binding ability of the pool of polyphages obtained in each round was enriched as the round progressed (FIG. 2a and FIG. 2b).

### Example 2-3: Positive phage screening (monophage ELISA)

Colonies obtained from a pool of three-round polyphages with high binding ability for antigen were inoculated in 1 ml of 2xYTCM (2% glucose, 5 mM MgCl₂) medium and placed in 96-well deep well plates (Bionia, 90030) and incubated for 16 hours in a 37°C incubator. 100-200 µl of the incubated cell cultures was taken to a value of 0.1 at OD600 and inoculated into 1 ml of 2xYTCM (2% glucose, 5 mM MgCl₂) medium, and incubated for 2-3 hours in a 37°C incubator. After incubation, M1 helper phage was added at an M value of 1:20 and inoculated into 2xYTCMK (5 mM MgCl₂, 1 mM IPTG) and incubated at 30°C for 16 hours.

A 96-well immune plate (NUNC, 439454) was coated with 100 ng/well of resistin antigen and blocked with skim milk. Monoclonal scFv phage, incubated for 16 hours, was added to each well at 100 µl for 2 hours, and each well was washed with PBS-T, and reacted with anti-M13-HRP (Amersham, 27-9421-01) at 1:2000 as a secondary antibody. After PBS-T washing, 100 µl of OPD (sigma, 8787-TAB) solution was applied to each well for 10 min of substrate reaction. After 10 minutes, the substrate reaction was stopped by treating each well with 50 µl of 1N H₂SO₄ and measured at 490 nm using a spectrophotometer (thermo fisher scientific, 51119200).

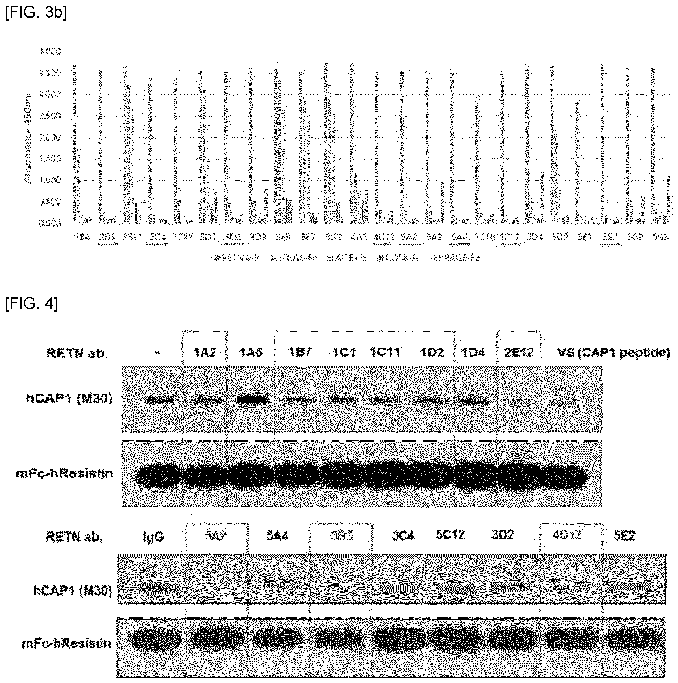

A total of 20 antigen-specific clones were obtained (FIG. 3a and FIG. 3b).

### Example 3: Production of primary screened resistin human antibodies

### Example 3-1: Conversion to IgG form

20 anti-resistin monoclonal phage antibodies screened in Example 2-3 were converted from the scFv construct to the IgG construct. To insert the DNA of each clone into the whole vector, PCR in which restriction enzyme sequences were added before and after each heavy and light chain was performed. The resulting heavy and light chain DNA and the entire vector were treated with restriction enzymes, followed by treatment with a ligase (T4 DNA ligase, thermofisher scientific, #EL0011) and a ligation reaction at 22°C for 10 minutes to insert the heavy and light chain sequences into the vector. The prepared vector was heat shock transfected into competent cells (XLI-blue) at 42°C and then spread on LB ampicillin flat medium and incubated at 37°C for at least 16 hours to obtain colonies. The obtained colonies were inoculated on LB ampicillin medium and incubated at 37°C for 16 hours. After the end of the incubation, the colony culture was collected and centrifuged at 3000 rpm to obtain the supernatant. DNA was extracted from the supernatant using a DNA prep kit (Nuclogen). The DNA sequence was analyzed to confirm that the cloning was successful.

### Example 3-2: Production of human antibody protein

The cloned heavy chain vector and light chain vector DNAs were co-transfected into HEK293F cells in a 6:4 (light chain : heavy chain) ratio. Vector DNA was mixed with polyethylenimine (PEI) to make a polyplex reaction and transfected into cells. Cultures were harvested on day 7 of transfection and proteins in the supernatant were purified by recombinant protein A agarose resin.

### Example 4: Characteristics of primary screened resistin human monoclonal antibodies

### Example 4-1: Antigen-receptor binding inhibition ability analysis

The antibodies were selected from a pool of antibody candidates by inhibiting the binding of CAP1 to resistin by an in vitro pull-down assay. A recombinant resistin protein Fc fused to THP-1 cell lysates was mixed with each antibody, and precipitated with Fc beads, and the bound CAP1 was measured by Western blotting to compare the binding of resistin and CAP1 and the binding inhibition effect of each antibody.

Specifically, THP-1 (ATCC TIP-202, Homo sapiens, Human) cells were obtained by cell line culturing methods of American Type Culture Collection (ATCC), the distributor. These cells were cultured in RPMI-1640 medium, pH 7.4, containing 100 units/ml penicillin, and 100 µg/ml streptomycin, in an incubator maintained at 37°C, CO₂ and 5% partial pressure. THP-1 cells were lysed with lysis buffer (20 mM Tris pH 7.5, 150 mM NaCl, 1% Triton X-100, 0.25% sodium deoxycholate, 1 mM EDTA, 1 mM NaF, 1 mM Na₃VO₄, and protease inhibitor cocktail) to extract protein, and 100 ng of mFc-hResistin (0.5 µg) protein and antibody were added to the extracted THP-1 protein (500 µg) and incubated overnight at 4°C. After adding mFc beads (20 µl) and pulling down at 4°C for 3 hours, it was washed three times with lysis buffer, then added with a SDS-PAGE sample buffer containing β-mercaptoethanol, boiled at 100°C for 5 minutes, and centrifuged to obtain supernatant. Whether the binding of hCAP1 and mFc-hResistin by VS peptide was inhibited was confirmed by Western blot using hCAP1 antibody and mFc-HRP antibody.

Antigen-receptor binding inhibition ability analysis of 16 proteins obtained from the primary screening yielded 5 highly effective proteins (1A2, 2E12, 5A2, 3B5, and 4D12) (FIG. 4).

### Example 4-2: Antigen affinity analysis

To investigate the affinity between the antibody and the resistin antigen, the Octet (Forte bio) system, which enables real-time investigation of ligand-receptor binding using a biosensor, was used. The system is an instrument that binds resistin antigen protein to a biosensor, flows antibody, and optically analyzes the degree of antibody binding to the antigen bound to the sensor, measuring the level of antibody binding to the antigen with high sensitivity at the light wavelength level. The affinity for resistin antigen was expressed as Kₒₙ (association constants, binding rate) and K_{dis} (dissociation constants, dissociation rate) between the receptor and ligand, and KD (equilibrium dissociation constant) for the reaction of binding and dissociation.

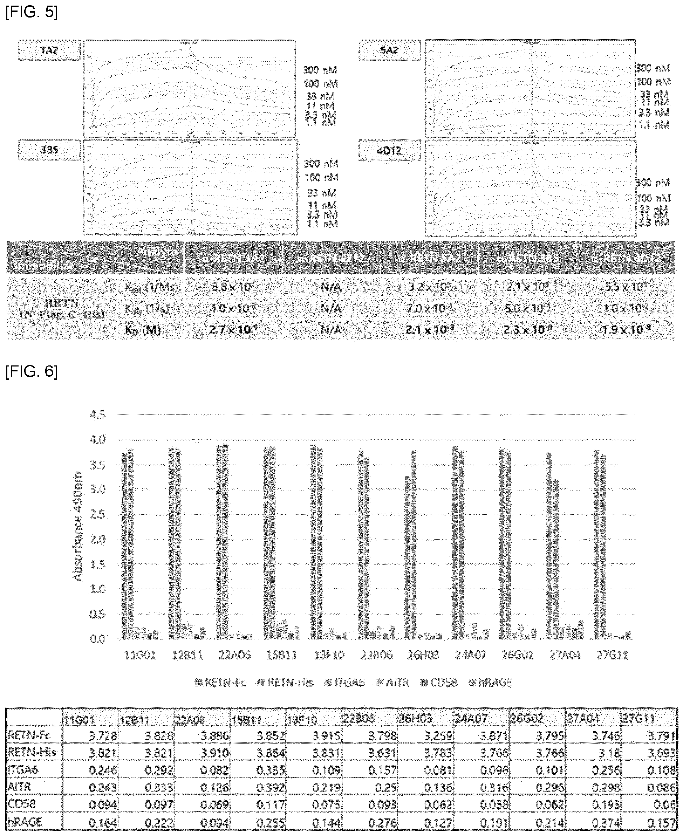

The results showed that among the 5 candidate antibodies 1A2, 2E12, 5A2, 3B5, and 4D12 that exhibited excellent effects in the antigen-receptor binding inhibition ability analysis, 4 clones, except 2E12, showed antigen affinity, with KDs as high as 1 nM (FIG. 5).

### Example 5: Secondary screening of resistin human antibodies

### Example 5-1: Biopanning

In addition to the primary screening, a secondary screening was performed to obtain candidate antibodies with high affinity for the antigen. The RETN-C-Fc or RETN-flag-His antigen produced in Example 1 was used, and the human antibody library phage was reacted with the antigen by coating an immunosorb tube as shown in Example 2, followed by elution of the scFV phage bound to the antigen. A total of three rounds of panning were performed, with each round yielding a pool of polyphages binding to the antigen.

### Example 5-2: Antigen-specific binding ability analysis of scFv-phaae antibodies

The scFv polyphage pool obtained from biopanning was cloned by monophage ELISA to obtain 84 scFv monoclones. To analyze their antigen-specific binding ability, 96-well immune plates (NUNC, 439454) were coated with resistin antigen and non-specific antigen at 100 ng/well and monoclonal scFv phages were reacted. The secondary antibody anti-M13-HRP (Amersham, 27-9421-01) was reacted, followed by a substrate reaction for 10 min with 100 µl of OPD (sigma, 8787-TAB) solution in each well. The substrate reaction was stopped with 1N H₂SO₄ and measured at 490 nm using a spectrophotometer (thermo fisher scientific, 51119200).

This resulted in acquire of 11 new clones with novel sequences and antigen-specific binding (FIG. 6).

### Example 6: Tertiary screening of resistin human antibodies

### Example 6-1: Biopanning

In addition to the first and second screening, a tertiary screening was performed to obtain candidate antibodies with high affinity for the antigen. The RETN-C-Fc or RETN-flag-His antigen produced in Example 1 was used, and the human antibody library phage was reacted with the antigen by coating an immunosorb tube with the antigen as shown in Example 2, followed by elution of the scFV phage bound to the antigen. A total of three rounds of panning were performed, with each round yielding a pool of polyphages binding to the antigen.

### Example 6-2: Antigen-specific binding ability analysis of scFv-phaae antibodies

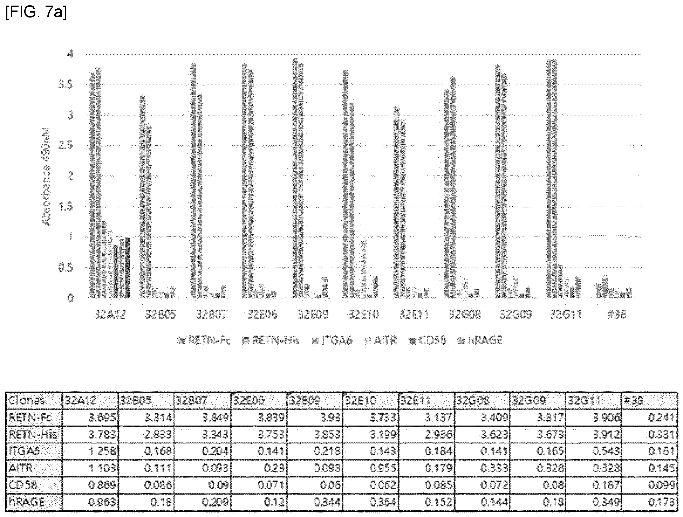

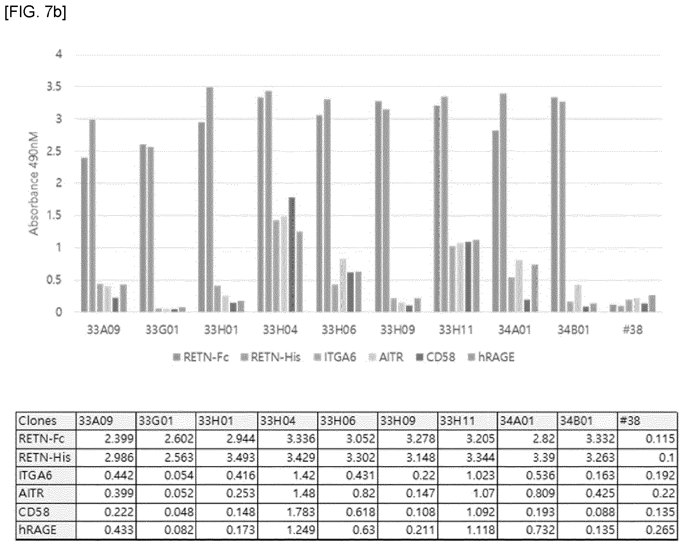

The scFv polyphage pool obtained from biopanning was cloned by monophage ELISA, resulting in 88 scFv monoclones. Their antigen-specific binding was analyzed by enzyme immunoassay, resulting in acquire of 10 new clones with novel sequences and antigen-specific binding (FIG. 7a and FIG. 7b).

### Example 7: Production of secondary and tertiary screened resistin human antibodies

### Example 7-1: Conversion to IgG form

A total of 20 monoclonal phage antibodies from the second and third screening were converted from scFv structure to IgG structure. As in Example 3, the DNA of each clone was inserted by ligation into the production vector and transformed into a competent cell (XLI-blue). Transformed cells were cultured in selection medium, and DNA was extracted therefrom and confirmed by sequencing.

### Example 7-2: Production of human antibody protein

The cloned heavy chain vector and light chain vector DNAs were co-transfected into HEK293F cells in a 6:4 (light chain : heavy chain) ratio. Cells were transfected by mixing polyethylenimine (PEI) and vector DNA to make a polyplex reaction, and soytone (BD, USA) was used as an enhancer. Cultures were harvested on day 7 of transfection and proteins in the supernatant were purified by recombinant protein A agarose resin.

### Example 8: Characteristics of secondary and tertiary screened resistin human monoclonal antibodies

### Example 8-1: Antigen-receptor binding inhibition ability analysis

The antibodies were selected from a pool of antibody candidates by inhibiting the binding of CAP1 to resistin by an in vitro pull-down assay. A recombinant resistin protein Fc fused to THP-1 cell lysates was mixed with each antibody, and precipitated with Fc beads, and the bound CAP1 was measured by Western blotting to compare the binding of resistin and CAP1 and the binding inhibition effect of each antibody.

Specifically, THP-1 (ATCC TIP-202, Homo sapiens, Human) cells were obtained by cell line culturing methods of American Type Culture Collection (ATCC), the distributor. These cells were cultured in RPMI-1640 medium, pH 7.4, containing 100 units/ml penicillin, and 100 µg/ml streptomycin, in an incubator maintained at 37°C, CO₂ and 5% partial pressure. THP-1 cells were lysed with lysis buffer (20 mM Tris pH 7.5, 150 mM NaCl, 1% Triton X-100, 0.25% sodium deoxycholate, 1 mM EDTA, 1 mM NaF, 1 mM Na₃VO₄, and protease inhibitor cocktail) to extract protein, and 100 ng of mFc-hResistin (0.5 µg) protein and antibody were added to the extracted THP-1 protein (500 µg) and incubated overnight at 4°C. After adding mFc beads (20 µl) and pulling down at 4°C for 3 hours, it was washed three times with lysis buffer, then added with a SDS-PAGE sample buffer containing β-mercaptoethanol, boiled at 100°C for 5 minutes, and centrifuged to obtain supernatant. Whether the binding of hCAP1 and mFc-hResistin by VS peptide was inhibited was confirmed by Western blot using hCAP1 antibody and mFc-HRP antibody.

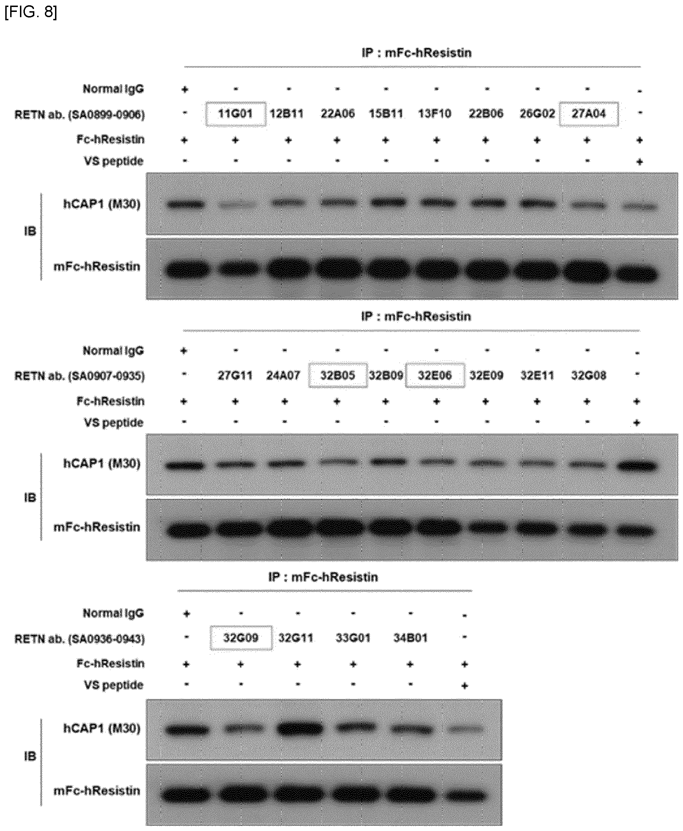

This resulted in 5 highly effective variants (11G01, 27A04, 32B05, 32E06, and 32G09) (FIG. 8).

### Example 8-2: Antigen-specific binding ability analysis

Enzyme-linked immunospecific assay (ELISA) was performed to investigate the specific binding of the screened clones to human resistin protein and to determine non-specific binding to other antigenic proteins. A 96-well immune plate (NUNC, 439454) was coated with the RETN-flag-His antigen prepared in Example 1 and non-specific antigen diluted in DPBS at 10 nM, 100 µl per well, and incubated at 4°C for 16 hours. It was then blocked with skim milk diluted 4% in PBS for 1 hour at 37°C. Antibody proteins were diluted in 1% skim milk that was diluted in PBS to be 1, 10, and 100 nM, respectively, at 100 µl per well and incubated for 2 hours at 37°C. After washing with PBS-T, anti-human Fc-HRP (Pierce(R) Peroxidase Conjugated Goat Anti-Human IgG FC (thermo fisher scientific, 31413)) as a secondary antibody was diluted to 1:10,000 and reacted at 37°C for 1 hour. After PBS-T washing, 100 µl of TMB (sigma, T0440) solution was treated to each well for a 10 min substrate reaction. After 10 minutes, the substrate reaction was stopped by treating each well with 50 µl of 1N H₂SO₄ and measured at 490 nm using a spectrophotometer (thermo fisher scientific, 51119200).

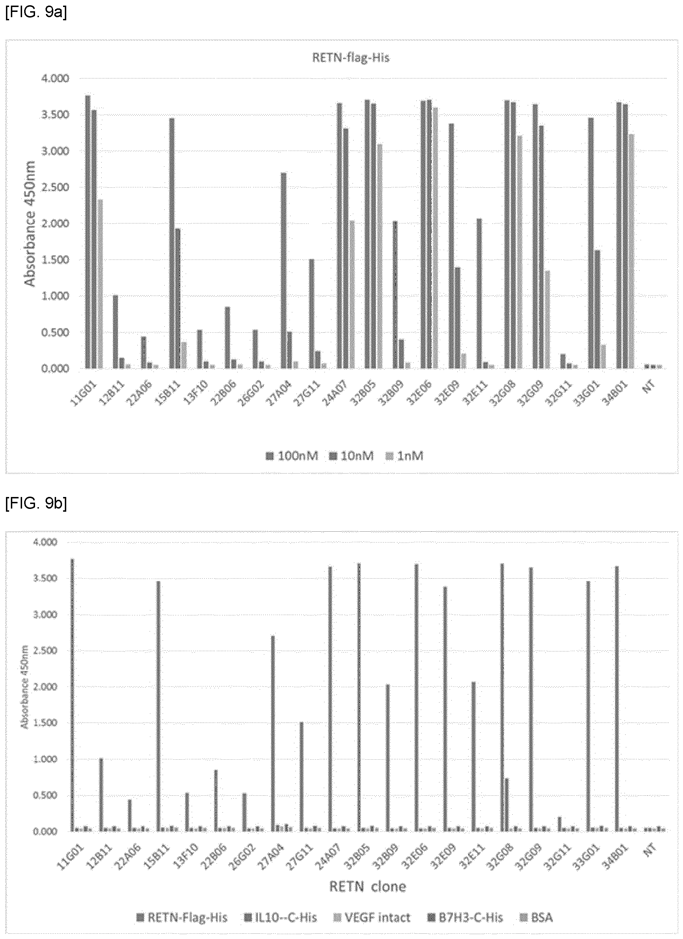

As a result, all 5 antibodies 11G01, 27A04, 32B05, 32E06, and 32G09 that exhibited excellent effects in the analysis of the antigen-receptor binding inhibition ability showed high specific binding ability to the RETN-flag-His antigen protein (FIG. 9a). In addition, all 5 antibodies that exhibited excellent effects in the analysis of the antigen-receptor binding inhibition ability compared to 5 nonspecific antigens did not show nonspecific binding (FIG. 9b).

### Example 8-3: Antigen affinity analysis

To investigate the affinity between the antibody and the resistin antigen, a ligand-receptor analysis was performed using the Octet (Forte bio) system biosensor.

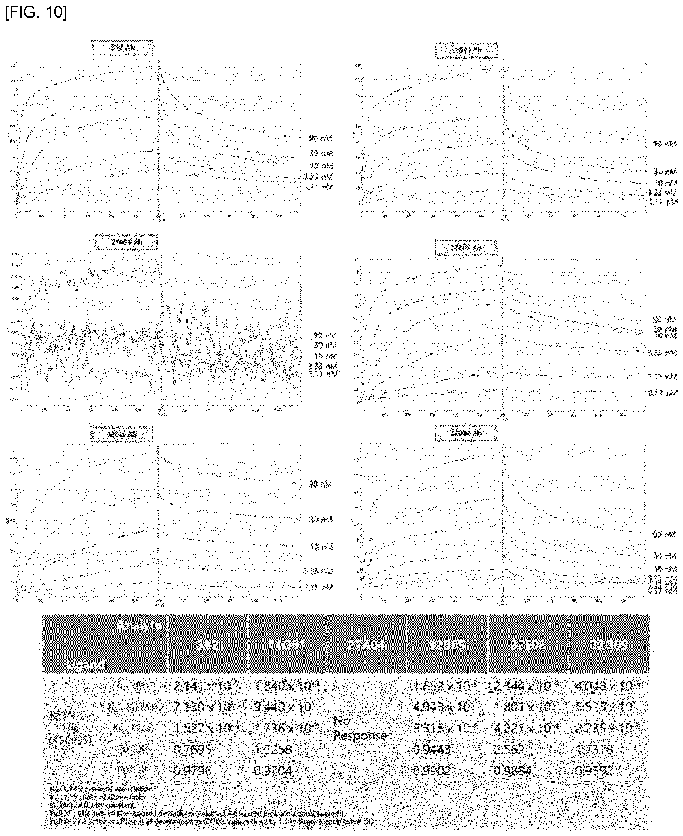

The results showed that 4 of the 5 candidate antibodies that exhibited excellent effects in the analysis of the antigen-receptor binding inhibition ability had antigen affinity with KDs as high as 1 nM (FIG. 10).

### Example 8-4: Anti-resistin leading antibody screening

The CDR sites of VH and VL of each clone were examined from the sequence analysis results of 40 monoclonal antibodies obtained from the first to third screening, and the similarity to the germ line antibody family was analyzed using the Ig BLAST program on the NCBI web page of http://www.ncbi.nlm.nih.gov/igblast/.

The results are shown in Table 7 below.

**[Table 7]**

| Similarity of germ line antibody family of 40 primary to tertiary screened clones | | | | |
|---|---|---|---|---|
| Clone name | Germ line VH | Similarity | Germ line VL | Similarity |
| RETN-1A1 | VH3-23 | 86.7% | A27 | 84.4% |
| RETN-1A2 | VH6-1 | 94.1% | L8 | 91.6% |
| RETN-1A3 | VH3-9 | 83.7% | V1-4 | 90.9% |
| RETN-1A6 | VH1-69 | 94.9% | V3-4 | 97.0% |
| RETN-1B7 | VH3-49 | 75.0% | L12a | 90.4% |
| RETN-1C1 | VH1-18 | 88.8% | V1-13 | 90.8% |
| RETN-1C11 | VH3-43 | 86.7% | L1 | 87.4% |
| RETN-1D2 | VH3-9 | 95.9% | L5 | 88.4% |
| RETN-1 D4 | VH3-74 | 82.5% | V1-2 | 95.9% |
| RETN-1D9 | VH3-15 | 89.0% | O1 | 95.0% |
| RETN-1F4 | VH1-46 | 87.8% | O1 | 97.0% |
| RETN-2E12 | VH3-33 | 84.7% | V1-13 | 90.8% |
| RETN-3B5 | VH3-23 | 88.8% | A20 | 89.5% |
| RETN-3C4 | VH1-69 | 84.7% | V1-16 | 86.0% |
| RETN-3D2 | VH3-30 | 91.8% | B3 | 89.1% |
| RETN-4D12 | VH1-46 | 88.8% | A18b | 94.0% |
| RETN-5A2 | VH1-69 | 89.8% | L8 | 87.4% |
| RETN-5A4 | VH1-46 | 90.8% | L8 | 96.8% |
| RETN-5C12 | VH3-9 | 89.9% | V2-1 | 89.5% |
| RETN-5E2 | VH1-69 | 81.6% | V2-14 | 82.8% |
| RETN-11G01 | VH3-21 | 92.8% | L19 | 86.3% |
| RETN-12B11 | VH3-48 | 87.8% | L1 | 91.6% |
| RETN-13F10 | VH3-23 | 82.7% | L19 | 87.4% |
| RETN-15B11 | VH3-30 | 90.8% | L8 | 87.4% |
| RETN-22A06 | VH3-49 | 94.0% | L19 | 87.4% |
| RETN-22B06 | VH3-15 | 90.9% | L19 | 87.4% |
| RETN-24A07 | VH3-11 | 90.8% | L19 | 90.5% |
| RETN-26G02 | VH3-15 | 90.8% | L19 | 87.4% |
| RETN-27A04 | VH3-73 | 87.0% | B3 | 87.1% |
| RETN-27G11 | VH3-43 | 85.9% | L19 | 85.3% |
| RETN-32B05 | IGHV1-69 | 88.8% | IGLV2-14 | 85.9% |
| RETN-32B09 | IGHV3-43D | 91.9% | IGKV1-39 | 88.4% |
| RETN-32E06 | IGHV3-30-5 | 86.7% | IGLV2-14 | 80.8% |
| RETN-32E09 | IGHV3-30 | 66.3% | IGKV1-9 | 85.3% |
| RETN-32E11 | IGHV3-21 | 83.7% | IGKV1-39 | 91.6% |
| RETN-32G08 | IGHV3-33 | 91.8% | IGKV1-12 | 92.6% |
| RETN-32G09 | IGHV3-74 | 87.8% | IGLV1-40 | 92.9% |
| RETN-32G11 | IGHV3-43D | 93.9% | IGKV1-17 | 90.5% |
| RETN-33G01 | IGHV3-9 | 80.8% | IGKV1-12 | 86.3% |
| RETN-34B01 | IGHV3-72 | 93.0% | IGKV1-27 | 86.3% |

### Example 9: Antibody optimization to resistin antibody RETN-5A2 clone

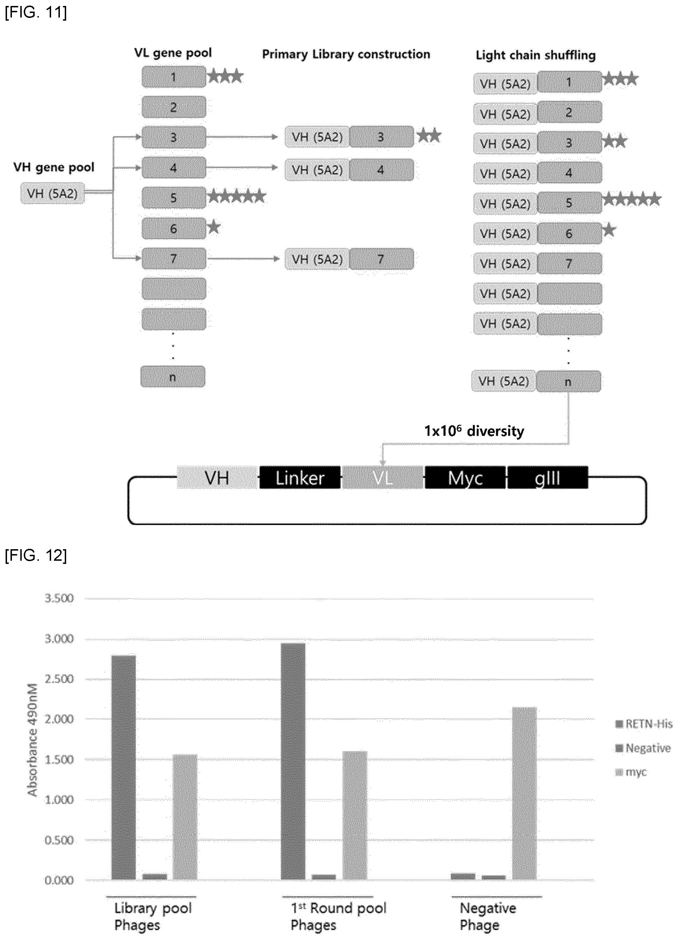

To optimize the antibody, the heavy chain of the RETN-5A2 clone was fixed and a light chain (LC) pool of 1×10⁶ diversity from Y BIOLOGICS was added to create a light chain shuffling library with a combination of a new light chain and the existing heavy chain. The light chain shuffling library was created by cutting the light chain sites of the RETN-5A2 gene with restriction enzymes to remove them, and then the 1×10⁶ diversity light chain pool was cut with the same restriction enzymes and ligated to the light chain-removed RETN-5A2 DNA. The cloned DNA was transformed into competent cells (XLI-blue), and the cells were pooled together to create a library (FIG. 11).

### Example 10: Screening of resistin human antibodies

### Example 10-1: Biopanning

To obtain an optimized phage pool using the 5A2 antibody that binds to the resistin antigen as the parent antibody, biopanning was performed. RETN-C-Fc or RETN-flag-His antigens prepared in Example 1 were coated onto immunosorb tubes and blocked. The 5A2 antibody optimized library phage was reacted with the antigen-coated immunosorb tubes, and the scFv phage bound to the antigen was eluted. The obtained phage was recovered and re-infected into *E. coli* for amplification.

### Example 10-2: Screening of positive phages and obtaining single antibody sequences

To obtain a phage-specific scFv pool for the antigen from the obtained scFv phage pool, a phage ELISA was performed. RETN-flag-His antigen was coated at 100 ng/well in a 96-well immune plate (NUNC, 439454) and blocked with skim milk. Each well was treated and reacted with the scFv phage obtained in each round, washed with PBS-T, and reacted with anti-M13-HRP (Amersham, 27-9421-01) at 1:2000 as a secondary antibody. After PBS-T washing, 100 µl of OPD (sigma, 8787-TAB) solution was applied to each well for 10 min of substrate reaction. After 10 minutes, the substrate reaction was stopped by treating each well with 50 µl of 1N H₂SO₄ and measured at 490 nm using a spectrophotometer (thermo fisher scientific, 51119200). The results showed a slight enrichment of the antigen-binding ability of the phage pool after biopanning (FIG. 12).

Colonies obtained from the first round polyphage pool with confirmed binding ability to antigen were inoculated in 1 ml of 2xYTCM (2% glucose, 5 mM MgCl₂) medium and placed in 96-well deep well plates (Bionia, 90030) and incubated for 16 hours in a 37°C incubator. 100-200 of the incubated cell cultures were taken to a value of 0.1 at OD600 and inoculated into 1 ml of 2xYTCM (2% glucose, 5 mM MgCl₂) medium and incubated for 2-3 hours in a 37°C incubator. After incubation, M1 helper phage was added at an M value of 1:20 and inoculated into 2xYTCMK (5 mM MgCl₂, 1 mM IPTG) and incubated at 30°C for 16 hours.

A 96-well immune plate (NUNC, 439454) was coated with 100 ng/well of resistin antigen and blocked with skim milk. Monoclonal scFv phage, incubated for 16 hours, was added to each well at 100 µl for 2 hours, and each well was washed with PBS-T, and reacted with anti-M13-HRP (Amersham, 27-9421-01) at 1:2000 as a secondary antibody. After PBS-T washing, 100 µl of OPD (sigma, 8787-TAB) solution was applied to each well for 10 min of substrate reaction. After 10 minutes, the substrate reaction was stopped by treating each well with 50 µl of 1N H₂SO₄ and measured at 490 nm using a spectrophotometer (thermo fisher scientific, 51119200).

As a result, a total of 36 antigen-specific clones were obtained, and the sequences of the 36 clones were analyzed to obtain 18 clones with unique sequences different from the existing sequences (Table 8).

**[Table 8]**

| Germ line antibody similarity of 18 optimized clones | | | | | |
|---|---|---|---|---|---|
| **Clone name** | **Light chain Isotype** | **Germ line VH** | **Similarity** | **Germ line VL** | **Similarity** |
| RETN-5A2_LS_1B11 | K | IGHV1-69 | 89.8% | IGKV3-11 | 89.5% |
| RETN-5A2_LS_1C10 | K | IGHV1-69 | 89.8% | IGKV1-12 | 93.7% |
| RETN-5A2_LS_1E04 | K | IGHV1-69 | 89.8% | IGKV1-12 | 90.5% |
| RETN-5A2_LS_1E09 | K | IGHV1-69 | 89.8% | IGKV1-12 | 88.4% |
| RETN-5A2_LS_1F09 | K | IGHV1-69 | 89.8% | IGKV1-8 | 91.6% |
| RETN-5A2_LS_1F11 | K | IGHV1-69 | 89.8% | IGKV4-1 | 94.1% |
| RETN-5A2_LS_1G11 | L | IGHV1-69 | 89.8% | IGLV1-44 | 87.8% |
| RETN-5A2_LS_2B02 | K | IGHV1-69 | 89.8% | IGKV3-20 | 86.5% |
| RETN-5A2_LS_2B10 | K | IGHV1-69 | 89.8% | IGKV1-5 | 87.4% |
| RETN-5A2_LS_2B12 | K | IGHV1-69 | 89.8% | IGKV1-39 | 87.4% |
| RETN-5A2_LS_2D07 | L | IGHV1-69 | 89.8% | IGLV2-14 | 86.9% |
| RETN-5A2_LS_2D09 | K | IGHV1-69 | 89.8% | IGKV1-5 | 91.6% |
| RETN-5A2_LS_2E10 | K | IGHV1-69 | 89.8% | IGKV1-33 | 85.3% |
| RETN-5A2_LS_2E11 | K | IGHV1-69 | 89.8% | IGKV1-5 | 85.3% |
| RETN-5A2_LS_2F11 | K | IGHV1-69 | 89.8% | IGKV1-39 | 90.5% |
| RETN-5A2_LS_2F12 | K | IGHV1-69 | 89.8% | IGKV1-9 | 88.4% |
| RETN-5A2_LS_2G03 | K | IGHV1-69 | 89.8% | IGKV1-39 | 89.5% |
| RETN-5A2_LS_2H10 | L | IGHV1-69 | 88.8% | IGLV2-14 | 84.8% |

### Example 11: Production of resistin human antibody

### Example 11-1: Conversion to IgG form

18 selected anti-resistin monoclonal phage antibodies were converted from the scFv structure to the IgG structure. To insert the DNA of each clone into the whole vector, PCR was performed in which restriction enzyme sequences were added before and after each heavy and light chain. The resulting heavy and light chain DNA and the entire vector were treated with restriction enzymes and with a ligase (T4 DNA ligase, thermofisher scientific, #EL0011), followed by a ligation reaction for 10 minutes at room temperature to insert the heavy and light chain sequences into the vector. The prepared vector was heat shock transfected into competent cells (XLI-blue) at 42°C and then spread on LB ampicillin flat medium and incubated at 37°C for at least 16 hours to obtain colonies. The obtained colonies were inoculated on LB ampicillin medium and incubated at 37°C for 16 hours. After the end of incubation, the colony culture was harvested and centrifuged at 3000 rpm to obtain the supernatant. DNA was extracted from the supernatant using a DNA prep kit (Nuclogen).

### Example 11-2: Production of human antibody protein

The cloned heavy chain vector and light chain vector DNA were co-transfected into HEK293F cells in a 6:4 (light chain : heavy chain) ratio. Vector DNA was mixed with polyethylenimine (PEI) to make a polyplex reaction and transfected into cells. On day 7 of transfection, the cultures were harvested and the proteins in the supernatant were purified with recombinant protein A agarose resin. The concentration of purified protein was quantified by measuring the absorbance at 280 nm using a nano-drop, and based on this, the productivity of each clone was evaluated (Table 9).

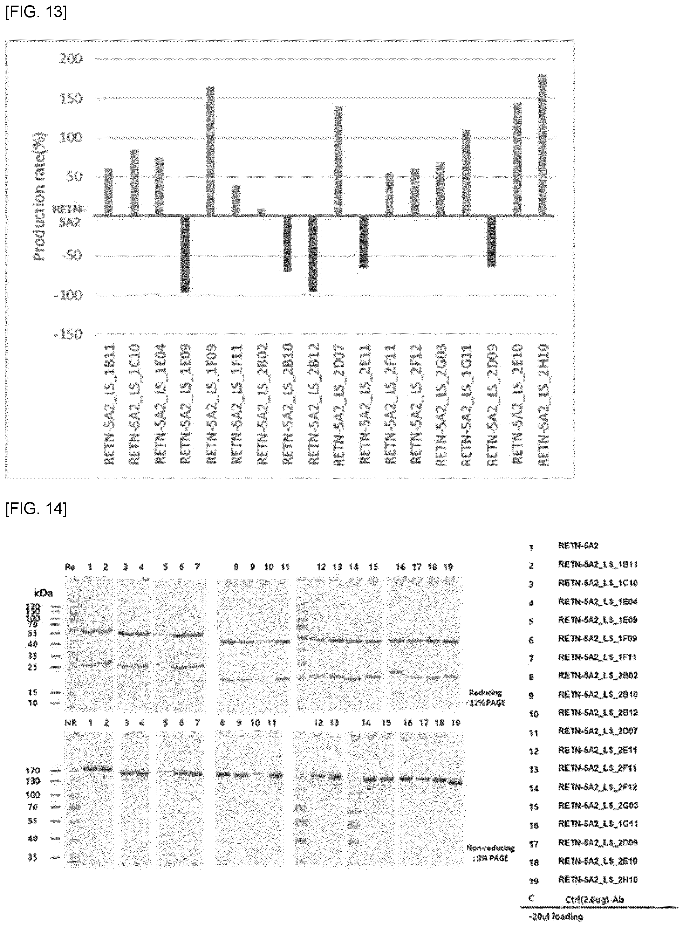

As a result, productivity increased by an average of 92% compared to the parent antibody 5A2 clone (FIG. 13).

**[Table 9]**

| Protein yield of 18 optimized antibodies | | | | |
|---|---|---|---|---|
| **NAME** | Conc. | Protein | Productivity | Increase rate compared to 5A2 |
| | [mg/ml] | [mg] | [mg/L] | (%) |
| **RETN-5A2** | 0.26 | 0.2 | 40 | |
| **RETN-5A2_LS_1B11** | 0.4 | 0.32 | 64 | 60 |
| **RETN-5A2_LS_1C10** | 0.51 | 0.37 | 74 | 85 |
| **RETN-5A2_LS_1E04** | 0.45 | 0.35 | 70 | 75 |
| **RETN-5A2_LS_1E09** | 0.03 | 0.007 | 1.4 | -96.5 |
| **RETN-5A2_LS_1F09** | 0.73 | 0.53 | 106 | 165 |
| **RETN-5A2_LS_1F11** | 0.37 | 0.28 | 56 | 40 |
| **RETN-5A2_LS_2B02** | 0.28 | 0.22 | 44 | 10 |
| **RETN-5A2_LS_2B10** | 0.08 | 0.06 | 12 | -70 |
| **RETN-5A2_LS_2B12** | 0.01 | 0.008 | 1.6 | -96 |
| **RETN-5A2_LS_2D07** | 0.52 | 0.48 | 96 | 140 |
| **RETN-5A2_LS_2E11** | 0.09 | 0.071 | 14.2 | -64.5 |
| **RETN-5A2_LS_2F11** | 0.37 | 0.31 | 62 | 55 |
| **RETN-5A2_LS_2F12** | 0.41 | 0.32 | 64 | 60 |
| **RETN-5A2_LS_2G03** | 0.41 | 0.34 | 68 | 70 |
| **RETN-5A2_LS_1G11** | 0.47 | 0.42 | 84 | 110 |
| **RETN-5A2_LS_2D09** | 0.09 | 0.072 | 14.4 | -64 |
| **RETN-5A2_LS_2E10** | 0.6 | 0.49 | 98 | 145 |
| **RETN-5A2_LS_2H10** | 0.74 | 0.56 | 112 | 180 |

Furthermore, the expression of proteins was evaluated by adding reduced and nonreduced proteins on SDS-PAGE to analyze the pattern of bands, thereby analyzing the purity and size of the produced proteins.

The results showed that all selected 18 antibodies were 150 kDa in size under nonreducing conditions, while under reducing conditions, two sizes of protein were detected which are expected to have a heavy chain of ~50 kDa and a light chain of ~25 kDa (FIG. 14).

### Example 12: Evaluation of characteristics of resistin human monoclonal antibody

### Example 12-1: Antigen-specific and non-specific binding (including ability to bind a human intact)

An enzyme-linked immunospecific assay (ELISA) was performed to determine the resistin antigen-specific binding of the resistin optimized antibody protein. RETN-flag-His antigen and non-specific antigen prepared in Example 1 were diluted to 10nM in DPBS, and added to a 96-well immune plate (NUNC, 439454) at 100 µl per well, and coated at 4°C for 16 hours. It was then blocked with skim milk diluted 4% in PBS for 1 hour at 37°C. Antibody proteins were diluted in 1% skim milk diluted in PBS to 1, 10, and 100 nM, respectively, at 100 µl per well and incubated for 2 hours at 37°C. After washing with PBS-T, anti-human Fc-HRP (Pierce(R) Peroxidase Conjugated Goat Anti-Human IgG FC (thermo fisher scientific, 31413)) as a secondary antibody was diluted to 1:10,000 and reacted at 37°C for 1 hour. After PBS-T washing, 100 µl of TMB (sigma, T0440) solution was applied to each well for a 10 min substrate reaction. After 10 minutes, the substrate reaction was stopped by treating each well with 50 µl of 1N H₂SO₄ and measured at 490 nm using a spectrophotometer (thermo fisher scientific, 51119200).

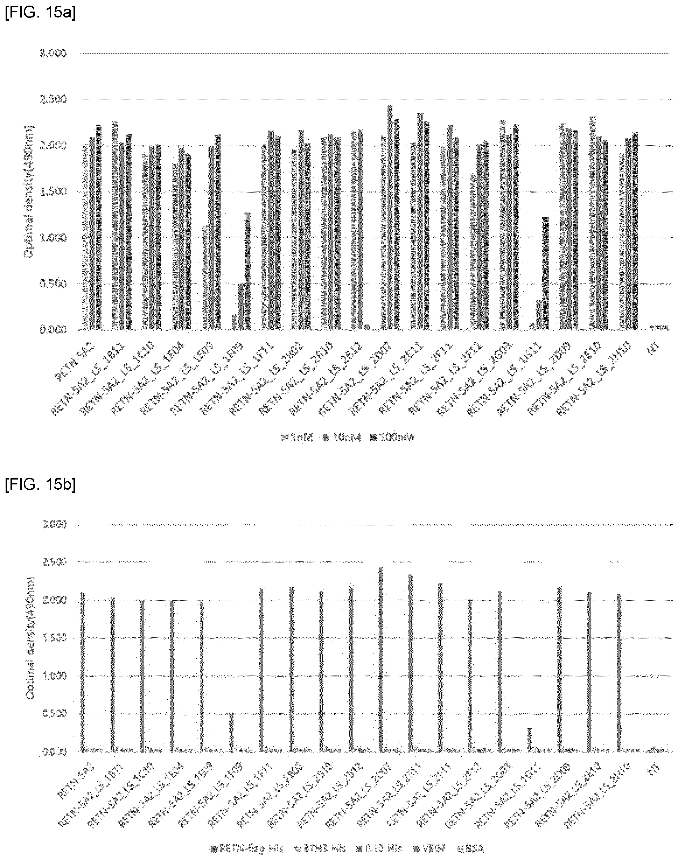

As a result, most of the clones except for a few clones (LS_1F09, LS_1G11) showed antigen binding reactions similar to 5A2 (FIG. 15a). In addition, all 18 optimized antibodies against the 5 non-specific antigens showed no non-specific binding (FIG. 15b).

The antigen produced in Example 1 has a human antibody Fc sequence or histidine sequence artificially added to the c-terminus of the antigen to facilitate purification and analysis. To confirm that the effect on the antigen in its natural state is the same as that of the antibody identified on the artificially structured antigen, an enzyme-linked immunospecific assay (ELISA) was performed using recombinant human antigen (recombinant human resistin, peprotech, #450-19) identical to the natural structure. 13 optimized antibodies were evaluated except for 5, i.e., LS_1E09, LS_2B10, LS_2B12, LS_2E11, and LS_2D09, which had significantly lower yields and resistin antigen binding than the parent antibody 5A2 clone. Recombinant human antigen (peprotech, #450-19) was diluted in DPBS at a concentration of 10 nM in 96-well immune plates (NUNC, 439454) at 100 µl per well and coated for 16 hours at 4°C. It was then blocked with skim milk diluted 4% in PBS for 1 hour at 37°C. Antibody proteins were diluted in 1% skim milk diluted in PBS to 1, 10, and 100 nM, respectively, at 100 µl per well and incubated for 2 hours at 37°C. After washing with PBS-T, it was incubated with anti-human Fc-HRP (Pierce(R) Peroxidase Conjugated Goat Anti-Human IgG FC (thermo fisher scientific, 31413)) at 1:10,000 dilution for 1 hour at 37°C as a secondary antibody. After PBS-T washing, 100 µl of TMB (sigma, T0440) solution was applied to each well for a 10 min substrate reaction. After 10 minutes, the substrate reaction was stopped by treating each well with 50 µl of 1N H₂SO₄ and measured at 490 nm using a spectrophotometer (thermo fisher scientific, 51119200).

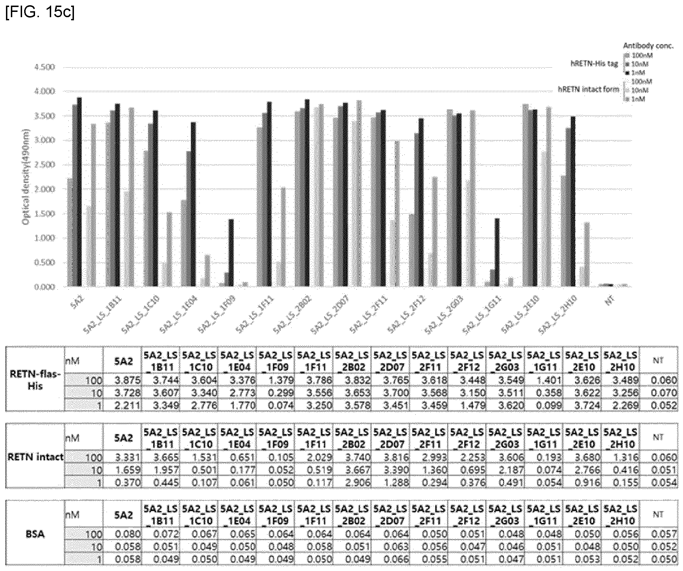

As a result, all clones, including the parent antibody 5A2, showed binding to intact form resistin in a manner very similar to the binding to conventional his tag resistin. From this, it is expected that the potency of the candidate clones against the natural antigen can be inferred (FIG. 15c).

### Example 12-2: Species cross-linking reaction to mouse antigen

An enzyme-linked immunospecific assay (ELISA) was performed to investigate the cross-species binding reactions of 8 optimized antibodies to mouse resistin antigen, except for 5 antibodies LS_1E09, LS_2B10, LS_2B12, LS_2E11, and LS_2D09, which had significantly lower yields and resistin antigen binding ability than the parent antibody 5A2 clone. The mouse antigen was murine resistin (#450-28) from peprotech. Mouse antigen was diluted to 10 nM in DPBS in 96-well immune plates (NUNC, 439454) at 100 µl per well, and coated for 16 hours at 4°C. It was then blocked with skim milk diluted 4% in PBS for 1 hour at 37°C. Antibody proteins were diluted in 1% skim milk diluted in PBS to 1, 10, and 100 nM, respectively, at 100 µl per well and incubated for 2 hours at 37°C. After washing with PBS-T, anti-human Fc-HRP (Pierce(R) Peroxidase Conjugated Goat Anti-Human IgG FC (thermo fisher scientific, 31413)) was diluted at 1:10,000 dilution and reacted for 1 hour at 37°C as a secondary antibody. After PBS-T washing, 100 µl of TMB (sigma, T0440) solution was applied to each well for a 10 min substrate reaction. After 10 minutes, the substrate reaction was stopped by treating each well with 50 µl of 1N H₂SO₄ and measured at 490 nm using a spectrophotometer (thermo fisher scientific, 51119200).

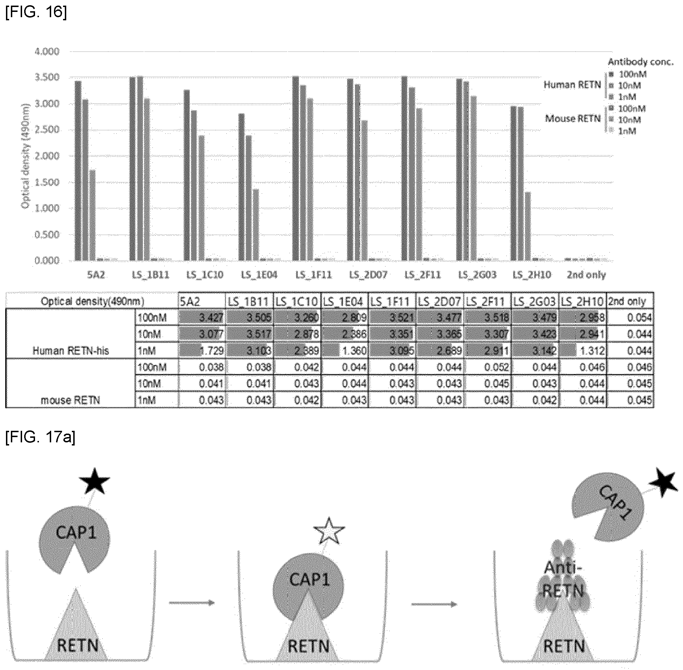

As a result, in contrast to the binding ability to the RETN-flag-His antigen, the parent antibody 5A2 clone and the 8 optimized antibodies did not bind to the mouse antigen. This confirmed that no cross-species binding reactions to mouse resistin occurred (FIG. 16).

### Example 12-3: Antigen-receptor binding inhibition ability analysis using an antigen-receptor competitive ELISA (resistin-CAP1 competitive ELISA)

To assess the efficacy of the antibody to bind to resistin and inhibit antigen-receptor binding, an antigen-receptor competitive enzyme-linked immunospecific assay (ELISA) was performed. A 96-well plate was coated with antigen protein, treated with antibody at different concentrations, and then treated with receptor to detect receptor protein bound to the antigen. A schematic of the assay is shown in FIG. 17a. The coated antigen protein was RETN-C-Fc protein prepared in Example 1, and the receptor protein was histidine-conjugated CAP1-His (LSbio, LS-G13682-100) which was purchased.

RETN-C-Fc protein diluted to 100 nM in DPBS was added to a 96-well immune plate (NUNC, 439454) at 100 µl per well, and it was coated for 16 hours at room temperature. It was then blocked with skim milk diluted 4% in PBS for 1 hour at 37°C. The antibody protein was serially diluted 5-fold from a peak concentration of 500 nM to 0.0064 nM, at 100 µl per well, and incubated for 2 hours at room temperature. After washing with PBS-T, CAP1-His was diluted to 100 nM in 1% skim milk diluted in PBS, 100 µl per well, and incubated for 2 hours at room temperature. After washing with PBS-T, anti-histidine-biotin (Invitrogen, MA1-21315-BTIN) was diluted to 1:2000 in 1% skim milk in PBS, at 100 µl per well, and reacted for 1 hour at room temperature, followed by streptavidin-HRP (thermo fisher scientific, 21140) diluted to 1:1000 and reacted for 1 hour at room temperature. After PBS-T washing, 100 µl of TMB (sigma, T0440) solution was applied to each well for a 10 min substrate reaction. After 10 minutes, the substrate reaction was stopped by treating each well with 50 µl of 1N H₂SO₄ and measured at 490 nm using a spectrophotometer (thermo fisher scientific, 51119200).

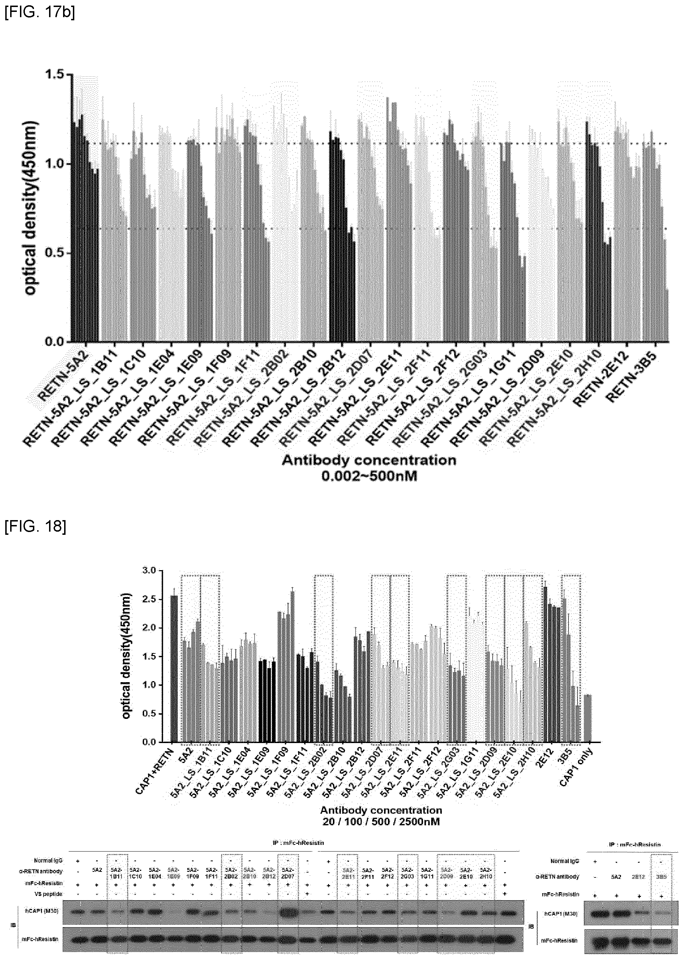

As a result, among the clones whose productivity and antigen binding ability were superior to parent antibody 5A2 in Example 11 and Example 12, the top 7 clones (5A2_LS_1F11, 5A2_LS_2B02, 5A2_LS_2D07, 5A2_LS_2F11, 5A2_LS_2G03, 5A2_LS_2E10, and 5A2_LS_2H10) with higher antigen-receptor binding inhibition ability than 5A2 were selected (FIG. 17b).

### Example 12-4: Antigen-receptor binding inhibition ability analysis

The antibodies were selected from a pool of antibody candidates by inhibiting the binding of CAP1 to resistin using an in vitro pull-down assay. THP-1 cell lysates were mixed with Fc-fused recombinant lysin protein and each antibody and precipitated with Fc beads, and the bound CAP1 was measured by Western blotting to compare the binding of resistin to CAP1 and the binding inhibition effect of each antibody.

Specifically, THP-1 (ATCC TIP-202, Homo sapiens, Human) cells were obtained by cell line culturing methods of American Type Culture Collection (ATCC), the distributor. THP-1 cells were cultured in RPMI-1640 medium, at pH 7.4, containing 100 units/ml penicillin, and 100 µg/ml streptomycin, in an incubator maintained at 37°C, CO₂ and 5% partial pressure. THP-1 cells were lysed with lysis buffer (20 mM Tris pH 7.5, 150 mM NaCl, 1% Triton X-100, 0.25% sodium deoxycholate, 1 mM EDTA, 1 mM NaF, 1 mM Na₃VO₄, and protease inhibitor cocktail) to extract protein, and 100 ng of mFc-hResistin (0.5 µg) protein and antibody were added to the extracted THP-1 protein (500 µg) and incubated overnight at 4°C. After adding mFc beads (20 µl) and pulling down at 4°C for 3 hours, it was washed three times with lysis buffer, then added with a SDS-PAGE sample buffer containing β-mercaptoethanol, boiled at 100°C for 5 minutes, and centrifuged to obtain supernatant. Whether the binding of hCAP1 and mFc-hResistin by VS peptide was inhibited was confirmed by Western blot using hCAP1 antibody and mFc-HRP antibody.

As a result, an antibody that was superior to the parent antibody 5A2 was obtained (FIG. 18).

### Example 12-5: investigation of affinity between antibody and resistin antigen using the Octet (Forte bio) system

To investigate the affinity between the antibody and the resistin antigen, the Octet (Forte bio) system, which enables real-time investigation of ligand-receptor binding using a biosensor, was used. The system is an instrument that binds resistin antigen protein to a biosensor, flows antibody through it, and optically analyzes the degree of antibody binding to the antigen bound to the sensor, measuring the level of antibody binding to the antigen with high sensitivity at the light wavelength level. The affinity for the resistin antigen was expressed as Kₒₙ (association constants, binding rate) and K_{dis} (dissociation constants, dissociation rate) between the receptor and ligand, and KD (equilibrium dissociation constant) for the reaction of binding and dissociation.

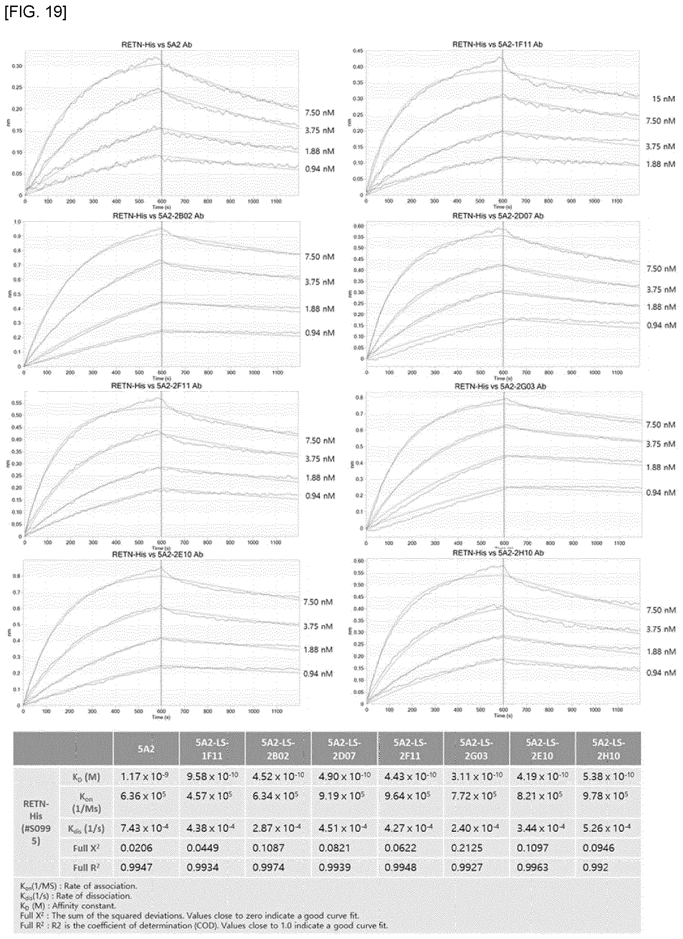

As a result, the 7 antibodies that outperformed the parent antibody in the antigen-receptor competitive enzyme-linked immunospecific assay had antigen affinities of KD 1 nM or less, which were equal to or better than parent antibody 5A2 (FIG. 19).

### Example 13: Analysis on the anti-cancer effect of resistin antibodies

### Example 13-1: NF-κB activation by resistin

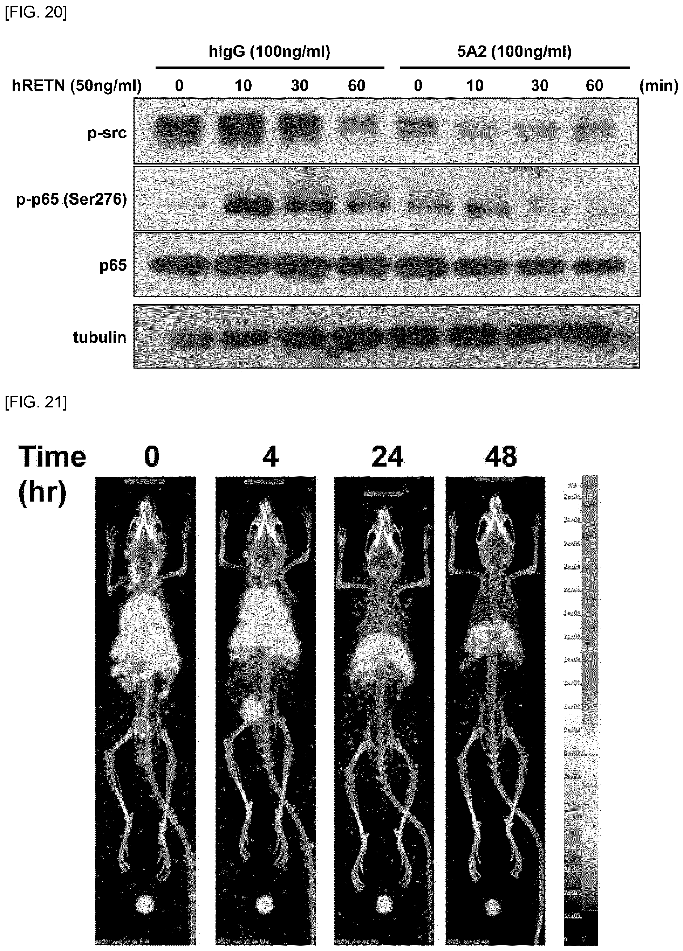

To determine the effect of the antibodies on the activity of NF-κB, when MB231 cells were transfected with resistin to activate NF-κB and added with 5A2 and IgG, the activity of NF-κB was measured by phosphorylation of p65 (FIG. 20).

Specifically, the MDA-MB-231 (ATCC HTB-26, Homo sapiens, Human) 293F cell line was cultured in DMEM medium, at pH 7.4, containing 100 units/ml penicillin, and 100 µg/ml streptomycin, in an incubator maintained at 37°C and 5% CO₂ partial pressure, using the cell line culture method of the American Type Culture Collection (ATCC), the distributor. 5.0 × 10⁵ MB-231 cells per well were inoculated into four 6-well plates and treated with 100 ng/ml anti-resistin_5A2 (Y-BIOLOGICS / ANRT) for 6 hours in 1% FBS-containing DMEM (Invitrogen Life Technologies) medium, followed by sequential treatment with 50 ng/ml recombinant human resistin (Y-BIOLOGICS / ANRT, Korea) for 10, 30, and 60 minutes.

For Western blot analysis, equal amounts of cell lysates were harvested and lysed in lysis buffer containing protease inhibitors (Roche, Cat.11836153001). Total protein (10 to 30 µg) was immunoblotted with specific primary antibodies; Phospho-Src (Tyr416) (Cell signaling Technology, #6943), p-p65 (Ser276) (Cell signaling Technology, #3037), total-p65 (Santa Cruz Biotechnology, sc-372), p-CREB (Ser133) (Santa Cruz Biotechnology, sc-101663), total-CREB (Cell signaling technology, #9197) and α-Tubulin (Calbiochem, Cat.CP06). Anti-mouse IgG HRP and anti-rabbit IgG HRP were purchased from Promega as secondary antibodies and detected with ECL and ECL-PLUS (Amersham).

### Example 13-2: Ex vivo cancer cell migration analysis

To determine the effect of antibodies on resistin-induced migration of cancer cells, cell migration experiments were performed on MB231 breast cancer cell line treated with 5A2 antibody and IgG.

5×10⁵ MDA-MB-231 breast cancer cells were cultured in 6 well plates. At 90% density, they were starved for 24 hours using RPMI1640 medium supplemented with 1% serum. Scratching was then induced using a 200p tip, followed by the addition of 100 ng/ml recombinant resistin, 100 ng/ml human normal IgG, and 100 ng/ml 5A2 antibody. After 6 hours, the migrated cells were observed using a Leica microscope (DMI3000 B) and quantified using Image J software based on the migrated area of the cells.

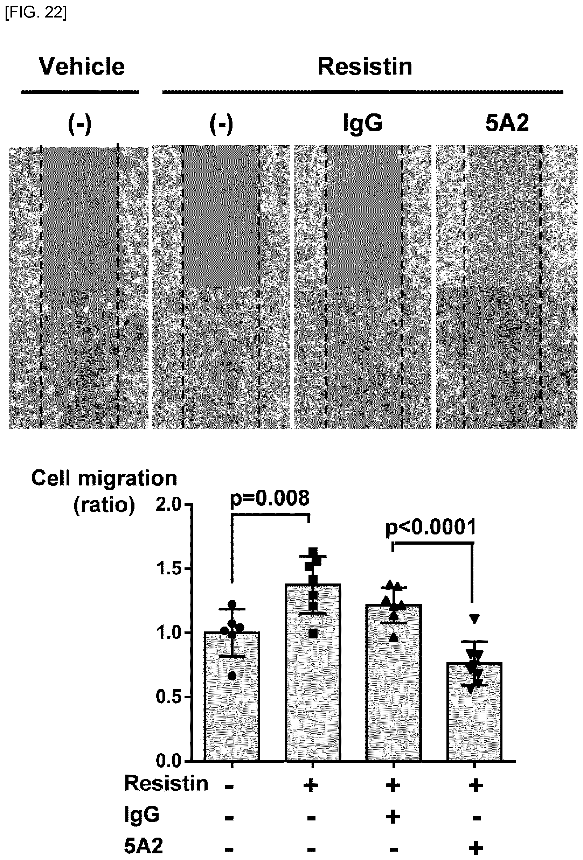

The results showed that 100 ng/ml resistin increased the migration of MDA-MB-231 breast cancer cells by approximately 137%. Human normal IgG, used as a negative control group, migrated breast cancer cells to a similar level as resistin alone, but co-treatment with 5A2, an anti-resistin antibody, inhibited cell migration by 55% compared to resistin alone and 76% compared to the vehicle group (FIG. 22).

### Example 13-3: Metastasis of cancer cells in vivo

To measure the pharmacokinetics, mice were administered with 5A2 antibody and hlgG control group to perform pharmacokinetic experiments (FIG. 21).

Furthermore, to confirm the metastasis inhibitory ability of 5A2 antibody against breast cancer cells in vivo, hyper-resistinmia was induced in NOD/SCID mice using adenovirus. It was confirmed by luminescence images and tissue harvesting that hyper-resistinmia promoted the metastasis of breast cancer cells to the liver and lungs. Specifically, 1x10⁷ pfu of Adv.GFP and Adv.hResistin were injected intraperitoneally (I.P.) into NOD/SCID mice, and one week later, 6×10⁵ luciferase-overexpressing MDA-MB-231 cells were injected intravenously (I.V.) to induce metastasis to the respective organs. Then, 10 mg/kg human normal IgG and 10 mg/kg 5A2 antibody were injected simultaneously, and were continuously administered at a frequency of 3 times per week thereafter. At week 4, luminescence images were obtained for each experimental group with administration of 150 mg/kg Luciferin. Images were acquired using a KODAK FX-PRO instrument.

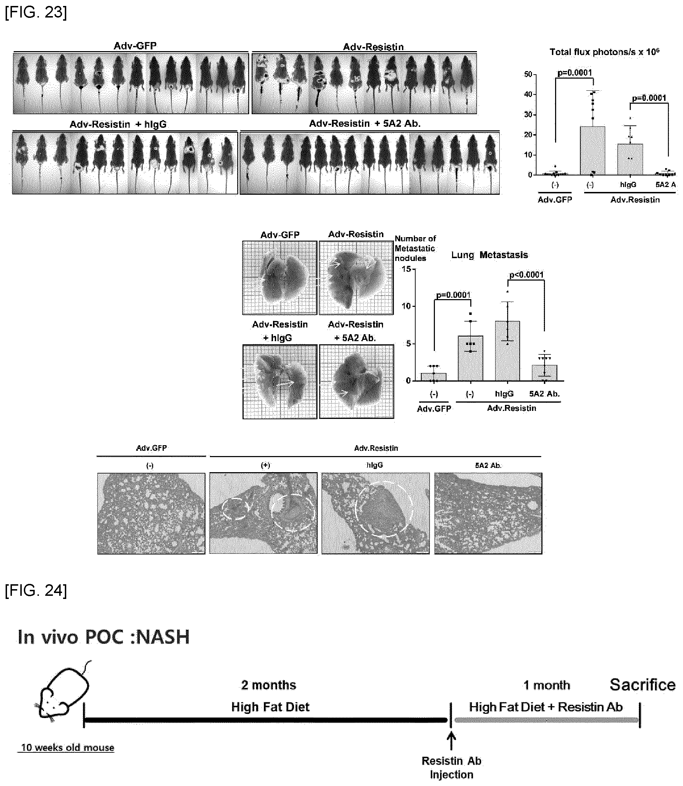

Administration with 10 mg/kg 5A2 antibody for 4 weeks (3 times per week) resulted in a significant reduction in metastasis to these organs. Furthermore, when the number of metastatic colonies that could be identified with the naked eye by harvesting the corresponding tissues was compared (arrows in FIG. 23), it was found that the number of metastatic colonies was significantly reduced in the 5A2 antibody group (FIG. 23), like in the luminescence image results.

### Example 14: Analysis on the effect of resistin antibodies on nonalcoholic steatohepatitis (NASH)

### Example 14-1: Confirmation of the effects of resistin antibodies in ameliorating high-fat diet-induced NASH

To determine whether the antibody ameliorates NASH induced by a high-fat diet, mice were induced with a high-fat diet for 2 months, fed a high-fat diet for 1 month, and given 5A2 by intravenous injection (I.V.) (FIG. 24).

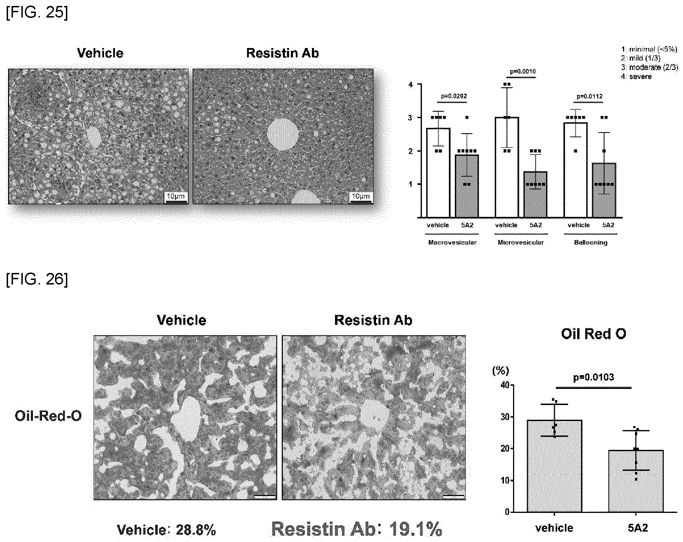

Mouse liver tissue was fixed in 4% PFA for 5 days, then paraffin blocked and stained with H&E, and the results were measured as nonalcoholic fatty liver disease activity score (NAS). The NAS score was significantly reduced in the 5A2 antibody group (FIG. 25).

Mouse liver tissue was made into OCT blocks and stained with Oil-Red-O, and the results showed that neutral triglycerides and lipids were significantly reduced in the 5A2 antibody group (FIG. 26).

### Example 14-2: Confirmation of insulin sensitivity enhancement by resistin antibodies

Insulin tolerance tests were performed 4 weeks after 5A2 administration. Mice were kept fasted and provided with water from 12 hours before testing. Before insulin administration, the tip of the mouse tail was cut and fasting blood glucose was measured using a Roche AccuCheck glucometer. Then, insulin 0.75 IU was administered by intraperitoneal injection (I.P.) and blood glucose was measured at 15, 30, 60 and 120 minutes.

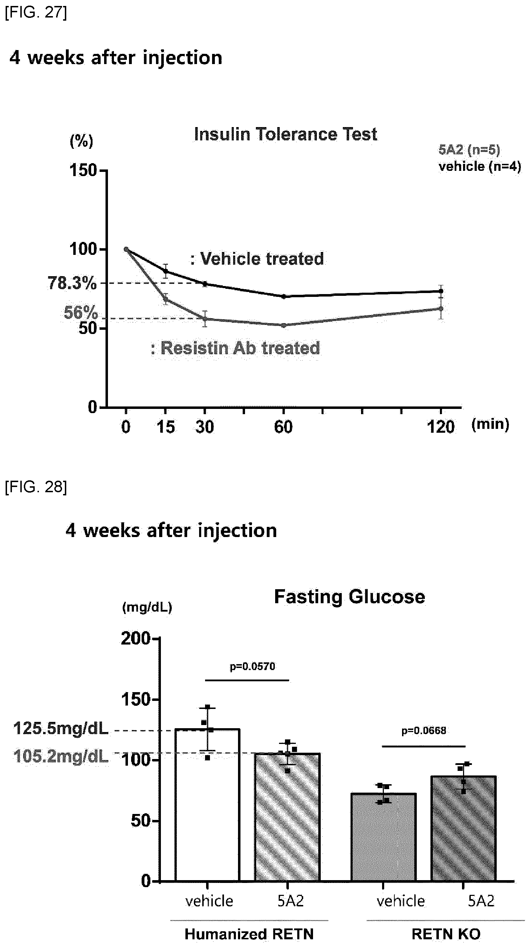

The results showed that insulin sensitivity was greater in the 5A2 antibody group, in particular, with a 56% reduction in fasting blood glucose at 30 minutes after insulin administration, which was 20% or more reduction from the control group (FIG. 27).

### Example 14-3: Confirmation of the effects of the resistin antibodies in reducing fasting glucose levels

Fasting blood glucose tests were performed 4 weeks after 5A2 administration. Mice were kept fasting for 12 hours and water was provided. Blood for blood glucose measurements was obtained by scissoring the tip of the mouse's tail and using a Roche AccuCheck glucose meter.

The results showed that humanized resistin mice overexpressing human resistin had a decrease in fasting blood glucose of 105.2 mg/dL, which was lower than that of the control group (125.5 mg/dL) (FIG. 28).

### Example 14-4: Confirmation of the LDL-cholesterol-lowering effect of resistin antibodies

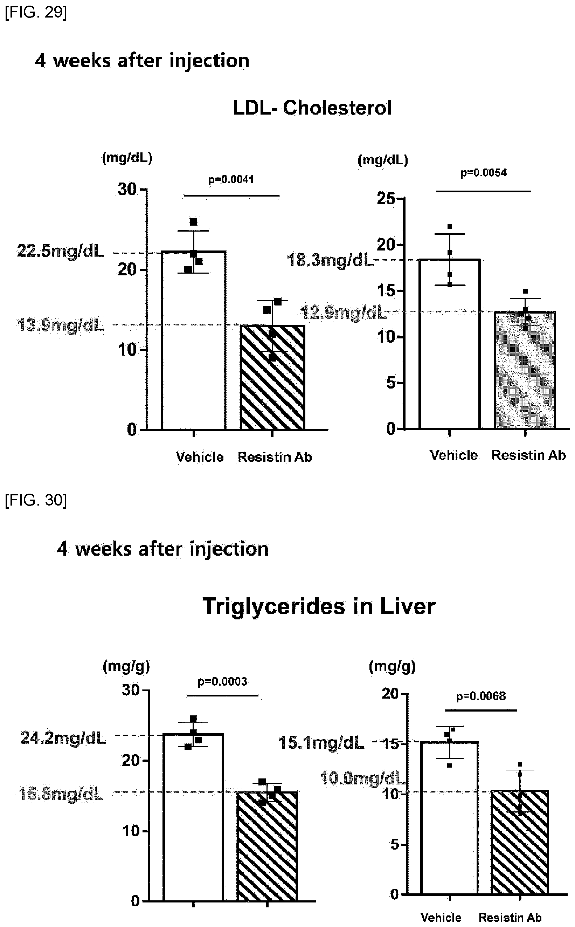

Mouse blood was collected and separated into serum using capillary blood collection tubes. The precipitation buffer provided by the cholesterol assay kit (ab65390) was mixed with an equal amount of serum, spun down at 13,000 rpm, and separated into supernatant (high-density lipoprotein) and pellet (low-density lipoprotein). The pellet was dissolved in PBS to obtain LDL. The results of two independent experiments showed that LDL was significantly reduced in the 5A2 antibody group compared to the control group (FIG. 29).

### Example 14-5: Confirmation of the effect of resistin antibodies in reducing triglyceride accumulation

50 mg of mouse liver was washed several times in cold PBS and homogenized in 1 ml of 5% NP-40/ddH20. It was then heated to 100°C to dissolve triglycerides. Triglycerides were measured using a triglyceride assay kit (ab65336) and a reduction of 30% or more was observed in the 5A2 antibody group compared to the control group (FIG. 30).

### Example 15: Analysis on the effect of resistin antibodies on inflammatory bowel disease (IBD)

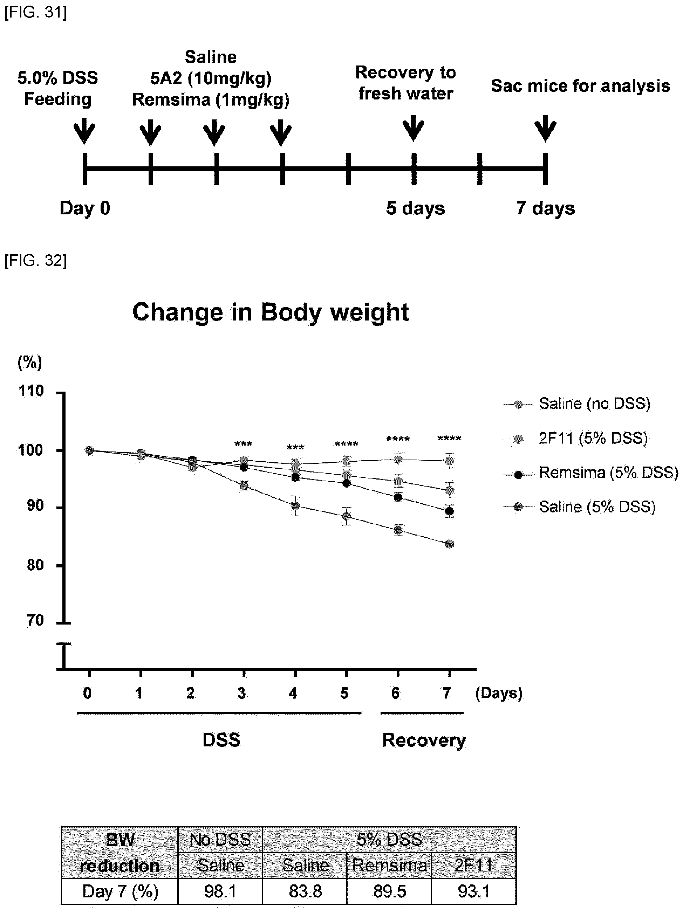

To determine if the antibody can ameliorate acute intestinal disease, intestinal disease was induced by watering with dextran sodium sulphate (DSS) at a concentration of 5% in drinking water for 5 days, followed by intravenous injection (I.V.) of saline, Remsima (1mg/kg), and RETN-5A2_LS_2F11 antibody (10mg/kg) on days 1-3 (FIG. 31).

Mouse weights were measured daily from the start of DSS administration and weight loss began to occur on day 3. Both Remsima and RETN-5A2_LS_2F11 caused less weight loss compared to the saline group, with RETN-5A2_LS_2F11 being more effective than Remsima by 3.6% (FIG. 32).

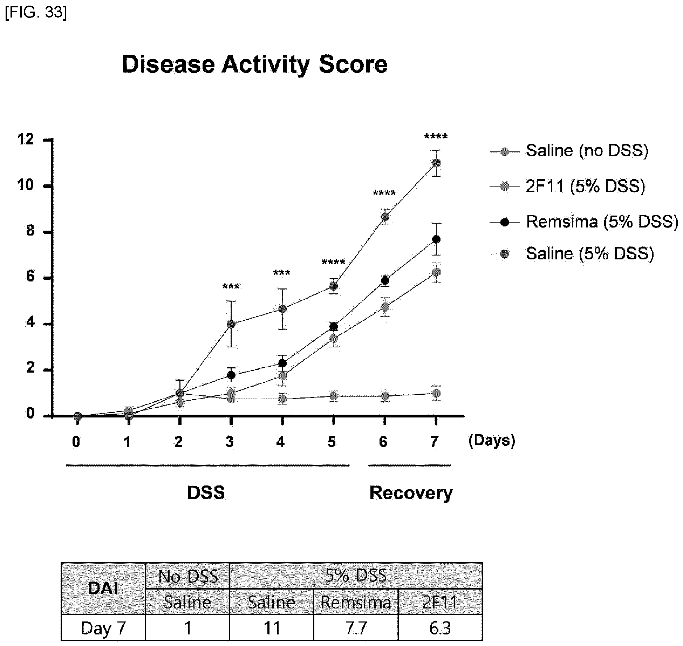

The disease activity score (DAI) is a combination of weight loss (%), stool quality, and bloody stools, which are scored on a scale of 0 to 4 and summed. DAI was measured daily from the start of DSS administration and began to increase on day 3. Both Remsima and RETN-5A2_LS_2F11 resulted in a smaller increase in DAI compared to the saline group, with RETN-5A2_LS_2F11 being more effective than remsima by 1.4 (FIG. 33).

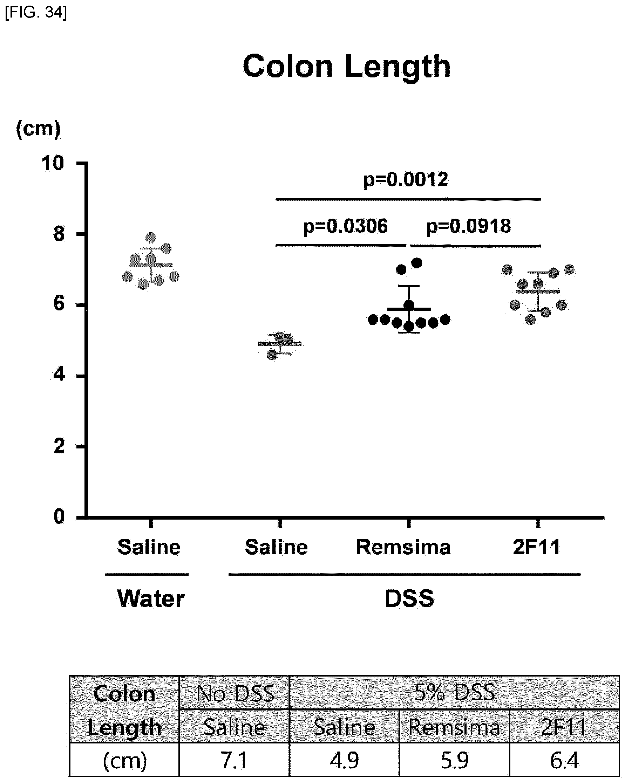

Mouse colon length is measured from the cecum to the anus. In the normal group, the intestinal length was 7.1 cm, which was reduced to 4.9 cm by DSS. Mouse intestinal length in both Remsima and RETN-5A2_LS_2F11 was reduced to a lesser extent compared to the saline group, with RETN-5A2_LS_2F11 showing more intestinal length protection than Remsima by 0.5 cm (FIG. 34).

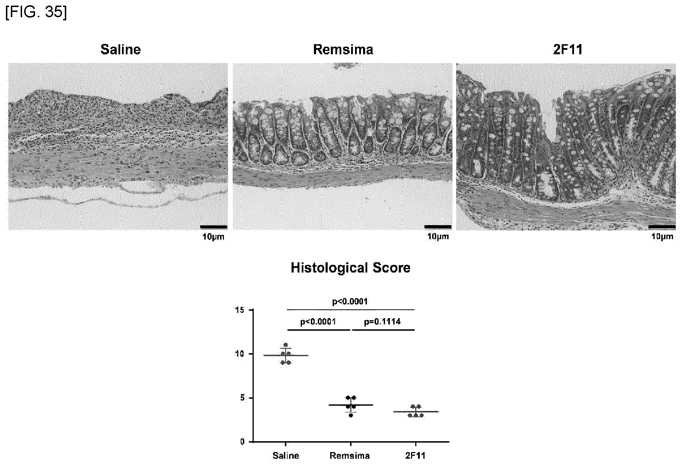

Mouse colon tissues were fixed in 4% PFA for 5 days, then paraffin blocked and stained with H&E, and the results were measured by histological score. Both Remsima and RETN-5A2_LS_2F11 caused a smaller increase in histological score compared to the saline group, with RETN-5A2_LS_2F11 being more effective than Remsima by 0.8 (FIG. 35).

### INDUSTRIAL APPLICABILITY

The novel antibody or antigen-binding fragment thereof that bind to resistin of the present invention can inhibit activity of resistin by blocking resistin/CAP1 binding and therefore are useful in the development of therapeutics for various diseases associated with resistin.

Although specific configurations of the present invention have been described in detail, those skilled in the art will appreciate that this description is provided to set forth preferred embodiments for illustrative purposes, and should not be construed as limiting the scope of the present invention. Therefore, the substantial scope of the present invention is defined by the accompanying claims and equivalents thereto.

### SEQUENCE LISTING FREE TEXT

An electronic file is attached.

## Claims

1. An antibody specifically binding to resistin or antigen-binding fragment thereof, comprising:
a heavy chain variable region comprising CDR1 comprising an amino acid sequence of SEQ ID NO: 1, CDR2 comprising an amino acid sequence of SEQ ID NO: 2, and CDR3 comprising an amino acid sequence of SEQ ID NO: 3; and
a light chain variable region comprising CDR1 comprising an amino acid sequence selected from the group consisting of SEQ ID NOs: 4 to 11, CDR2 comprising an amino acid sequence selected from the group consisting of SEQ ID NOs: 12 to 19, and CDR3 comprising an amino acid sequence selected from the group consisting of SEQ ID NOs: 20 to 27.

2. The antibody specifically binding to resistin or antigen-binding fragment thereof according to claim 1, wherein the light chain variable region is:
a light chain variable region comprising CDR1 comprising the amino acid sequence of SEQ ID NO: 4, CDR2 comprising the amino acid sequence of SEQ ID NO: 12, and CDR3 comprising the amino acid sequence of SEQ ID NO: 20;
a light chain variable region comprising CDR1 comprising the amino acid sequence of SEQ ID NO: 5, CDR2 comprising the amino acid sequence of SEQ ID NO: 13, and CDR3 comprising the amino acid sequence of SEQ ID NO: 21;
a light chain variable region comprising CDR1 comprising the amino acid sequence of SEQ ID NO: 6, CDR2 comprising the amino acid sequence of SEQ ID NO: 14, and CDR3 comprising the amino acid sequence of SEQ ID NO: 22;
a light chain variable region comprising CDR1 comprising the amino acid sequence of SEQ ID NO: 7, CDR2 comprising the amino acid sequence of SEQ ID NO: 15, and CDR3 comprising the amino acid sequence of SEQ ID NO: 23;
a light chain variable region comprising CDR1 comprising the amino acid sequence of SEQ ID NO: 8, CDR2 comprising the amino acid sequence of SEQ ID NO: 16, and CDR3 comprising the amino acid sequence of SEQ ID NO: 24;
a light chain variable region comprising CDR1 comprising the amino acid sequence of SEQ ID NO: 9, CDR2 comprising the amino acid sequence of SEQ ID NO: 17, and CDR3 comprising the amino acid sequence of SEQ ID NO: 25;
a light chain variable region comprising CDR1 comprising an amino acid sequence of SEQ ID NO: 10, CDR2 comprising an amino acid sequence of SEQ ID NO: 18, and CDR3 comprising an amino acid sequence of SEQ ID NO: 26; or
a light chain variable region comprising CDR1 comprising an amino acid sequence of SEQ ID NO: 11, CDR2 comprising an amino acid sequence of SEQ ID NO: 19, and CDR3 comprising an amino acid sequence of SEQ ID NO: 27.

3. The antibody specifically binding to resistin or antigen-binding fragment thereof according to claim 1, wherein the heavy chain variable region comprises an amino acid sequence of SEQ ID NO: 28.

4. The antibody specifically binding to resistin or antigen-binding fragment thereof according to claim 2, wherein the light chain variable region comprises an amino acid sequence selected from the group consisting of SEQ ID NOs: 32, 34, 36, 38, 40, 42, 44 and 46.

5. The antibody specifically binding to resistin or antigen-binding fragment thereof according to claim 1, which comprise a heavy chain variable region comprising an amino acid sequence of SEQ ID NO: 28; and
a light chain variable region comprising an amino acid sequence selected from the group consisting of SEQ ID NOs: 32, 34, 36, 38, 40, 42, 44 and 46.

6. A nucleic acid encoding a heavy chain variable region of the antibody or antigen-binding fragment thereof according to claim 1.

7. The nucleic acid according to claim 6, wherein the nucleic acid comprises a sequence of SEQ ID NO: 30.

8. A nucleic acid encoding a light chain variable region of the antibody or antigen-binding fragment thereof according to claim 1.

9. The nucleic acid according to claim 8, wherein the nucleic acid comprises a sequence selected from the group consisting of SEQ ID NOs: 48, 50, 52, 54, 56, 58, 60 and 62.

10. An expression vector comprising the nucleic acid encoding the heavy chain variable region of the antibody or antigen-binding fragment thereof according to claim 6, and the nucleic acid encoding the light chain variable region of the antibody or antigen-binding fragment thereof according to claim 8.

11. A cell transformed with the expression vector according to claim 10.

12. The cell according to claim 11, which is selected from the group consisting of animal cells, plant cells, yeast, *E. coli,* and insect cells.

13. A method of producing an antibody that specifically binds to resistin or antigen-binding fragment thereof, comprising the following steps
(a) culturing the cells according to claim 11; and
(b) recovering the antibody or antigen-binding fragment thereof from the cell culture.

14. An antibody-drug conjugate (ADC), in which a drug is conjugated to the antibody or antigen-binding fragment thereof according to claim 1.

15. The antibody-drug conjugate according to claim 14, wherein the antibody or antigen-binding fragment thereof are linked to a drug via a linker.

16. The antibody-drug conjugate according to claim 15, wherein the linker is a cleavable linker or a non-cleavable linker.

17. The antibody-drug conjugate according to claim 14, wherein the drug is a chemotherapeutic agent, toxin, micro RNA (miRNA), siRNA, shRNA, or radioisotope.

18. A bispecific or multi-specific antibody comprising the antibody or antigen-binding fragment thereof according to claim 1.

19. A chimeric antigen receptor (CAR) comprising the antibody or antigen-binding fragment thereof according to claim 1.

20. An immune cell comprising the chimeric antigen receptor according to claim 19.

21. The immune cell according to claim 20, wherein the immune cell is a T cell or an NK cell.

22. A pharmaceutical composition for the prevention or treatment of a disease that can be treated by inhibiting an activity of resistin comprising the antibody or antigen-binding fragment thereof according to claim 1, the antibody-drug conjugate according to claim 14, the bispecific or multi-specific antigen receptor according to claim 18, or the chimeric antigen receptor according to claim 19.

23. The pharmaceutical composition according to claim 22, wherein the activity of resistin is inhibited by blocking resistin/CAP1 binding by the resistin antibody.

24. The pharmaceutical composition according to claim 22, wherein the disease that can be treated by inhibiting the activity of resistin is cancer, cardiovascular metabolic disease, autoimmune disease, or inflammatory disease.

25. The pharmaceutical composition according to claim 24, wherein the cancer is selected from the group consisting of breast cancer, metastatic cancer, uterine cancer, ovarian cancer, prostate cancer, melanoma, lung cancer, liver cancer, glioblastoma, colorectal cancer, head and neck cancer, bladder cancer, renal cell carcinoma, gastric cancer, pancreatic cancer, and recurrent cancer.

26. The pharmaceutical composition according to claim 24, wherein the cardiovascular metabolic disease is selected from the group consisting of obesity, hyperlipidemia, hypertension, atherosclerosis, hyperinsulinemia, insulin resistance diabetes, type 2 diabetes, liver disease, non-alcoholic fatty liver disease, and non-alcoholic steatohepatitis.

27. The pharmaceutical composition according to claim 24, wherein the autoimmune disease is selected from the group consisting of chronic heart disease, rheumatoid arthritis, systemic lupus erythematosus, digestive diabetes, atopic dermatitis, autoimmune encephalomyelitis, asthma, and Crohn's disease.

28. The pharmaceutical composition according to claim 24, wherein the inflammatory disease is selected from the group consisting of inflammatory skin diseases such as asthma, eczema, psoriasis, acne, allergies, rheumatoid arthritis, psoriatic arthritis, atopic dermatitis, atopic rhinitis, allergic dermatitis, chronic sinusitis, or seborrheic dermatitis; inflammatory bowel disease (IBD) such as Crohn's disease or ulcerative colitis; and acute or chronic inflammatory diseases such as ankylosing spondylitis, sepsis, septic shock, vasculitis, and bursitis.
